# EUROPEAN PATENT APPLICATION

(11) **EP 2 292 666 A2**
(43) Date of publication of application: **09.03.2011**
(21) Application number: 10182277.3
(22) Date of filing: 10.03.2003
(51) Int. Cl.: C07K 16/30, A61K 39/395, A61K 47/48, A61K 51/10, A61P 35/00, C12N 5/28, C12N 15/08, G01N 33/574, G01N 33/577

(54) **Neoplasm specific antibodies and uses thereof**

(30) Priority: 09.03.2002 DE 10210427
(62) Divisional of application: 03710116.9
(71) Applicant: Patrys Limited, Melbourne, Victoria 3000 (AU)
(72) Inventor: Vollmers, Heinz Peter, 97078 Würzburg (DE); Mueller-Hermelink, Hans Konrad, 97082 Würzburg (DE)
(74) Representative: Cohausz & Florack

(57) **Abstract**

The present invention features polypeptides, such as embodiments, and their use in the treatment and diagnosis of neoplasms.

SEQUENCE LISTING

<110>  PATRYS PTY LIMITED
       VOLLMERS, HEINZ P
       MUELLER-HERMELINK, HANS K

<120>  NEOPLASM SPECIFIC ANTIBODIES AND USES THEREOF

<130>  043043-0389996

<140>  To be assigned
<141>  Herewith

<150>  DE 102 10 427.1
<151>  2002-03-09

<150>  PCT/IB2003/01335
<151>  2003-03-10

<160>  4

<170>  PatentIn version 3.3

<210>  1
<211>  102
<212>  PRT
<213>  Homo sapiens


<220>
<221>  misc_feature
<223>  Heavy chain CM-1 variable region

<400>  1

Arg Ser Leu Arg Leu Ser Cys Ala Ala Ser Gly Phe Thr Phe Ser Ser
1               5                   10                  15


Tyr Gly Met His Trp Val Arg Gln Ala Pro Gly Lys Gly Leu Glu Trp
            20                  25                  30


Val Ala Val Ile Ser Tyr Asp Gly Ser Asn Lys Tyr Tyr Ala Asp Ser
        35                  40                  45


Val Lys Gly Arg Phe Thr Ile Ser Arg Asp Asn Ser Lys Asn Thr Leu
    50                  55                  60


Tyr Leu Gln Met Asn Ser Leu Arg Ala Glu Asp Thr Ala Val Tyr Tyr
65                  70                  75                  80


Cys Ala Lys Asp Arg Ser Ser Gly Tyr Tyr Gly Met Asp Val Trp Gly
                85                  90                  95


Gln Gly Thr Leu Val Thr
            100


<210>  2
<211>  306
<212>  DNA
<213>  Homo sapiens

<220>
<221>  misc_feature
<223>  Heavy chain CM-1 variable region

<400>  2
aggtccctga gactctcctg tgcagcctct ggattcacct tcagtagcta tggcatgcac      60

tgggtccgcc aggctccagg caaggggctg gagtgggtgg cagttatatc atatgatgga     120

agtaataaat actatgcaga ctccgtgaag ggccgattca ccatctccag agacaattcc     180

aagaacacgc tgtatctgca aatgaacagc ctgagagctg aggacacggc tgtgtattac     240

tgtgcgaaag accggtcttc gggctactac ggtatggacg tctggggcca aggcaccctg     300

gtcacc                                                                306


<210>  3
<211>  109
<212>  PRT
<213>  Homo sapiens


<220>
<221>  misc_feature
<223>  Light chain CM-1 variable region

<400>  3

Ser Tyr Val Leu Thr Gln Pro Pro Ser Val Ser Val Ser Pro Gly Gln
1               5                   10                  15


Thr Ala Arg Ile Thr Cys Ser Gly Asp Ala Leu Pro Lys Gln Tyr Ala
            20                  25                  30


Tyr Trp Tyr Gln Gln Lys Pro Gly Gln Ala Pro Val Leu Val Ile Tyr
        35                  40                  45


Lys Asp Ser Glu Arg Pro Ser Gly Ile Pro Glu Arg Phe Ser Gly Ser
    50                  55                  60


Ser Ser Gly Thr Thr Val Thr Leu Thr Ile Ser Gly Val Gln Ala Glu
65                  70                  75                  80


Asp Glu Ala Asp Tyr Tyr Cys Gln Ser Ala Asp Ser Ser Gly Thr Tyr
                85                  90                  95


Val Val Phe Gly Gly Gly Thr Lys Leu Thr Val Leu Gly
            100                 105


<210>  4
<211>  327
<212>  DNA
<213>  Homo sapiens


<220>
<221>  misc_feature
<223>  Light chain CM-1 variable region

<400>  4

tcctatgtgc tgactcagcc accctcggtg tcagtgtccc caggacagac ggccaggatc 60
acctgctctg gagatgcatt gccaaagcaa tatgcttatt ggtaccagca gaagccaggc 120
caggcccctg tgctggtgat atataaagac agtgagaggc cctcagggat ccctgagcga 180
ttctctggct ccagctcagg gacaacagtc acgttgacca tcagtggagt ccaggcagaa 240
gacgaggctg actattactg tcaatcagca gacagcagtg gtacttatgt ggtattcggc 300
ggagggacca agctgaccgt cctaggt 327

## Description

### Background of the Invention

The present invention is related to the field of cancer diagnosis and treatment and, more specifically, to the identification of polypeptides, such as antibodies, useful in the diagnosis, detection, monitoring, and treatment of neoplasms in a mammal, e.g., a human.

In the United States well over one million individuals are diagnosed with cancer each year. Although recent advances in the medical field have significantly improved the rate of survival among cancer patients, a large number of cancer-related deaths still could be prevented by the early diagnosis of the tumor. Accordingly, at the time of initial diagnosis, an alarming number of patients have already reached late stages of the disease.

With respect to colorectal cancer, the prognosis is usually poor in 50% of all cases because the tumor is often undetected until the disease has spread and reached a terminal stage. Similarly, approximately 75% of women are diagnosed with ovarian cancer after the disease has already reached an advanced stage (stage III or IV) because the symptoms of ovarian cancer are often vague or "silent." Despite aggressive surgical intervention and new chemotherapeutic regimens, the overall 5-year survival rate for these women with advanced stage ovarian cancer has remained constant over the past 30 years, at approximately 15%. Conversely, women diagnosed with cancer confined to the ovary (stage I) have an overall 5-year survival rate approaching 90%.

Clearly, there is a need for the early and improved detection and treatment of neoplasms (e.g., a colorectal adenocarcinoma, ovarian cancer, squamous cell lung carcinoma, lobular mammary carcinoma, stomach carcinoma, esophagial squamous cell carcinoma, pancreatic adenocarcinoma, lung adenocarcinoma, ductal mammary carcinoma, uterine adenocarcinoma, or prostate adenocarcinoma), as this would increase the chance of treating the neoplasm and, thereby, lead to an improved prognosis for long-term survival.

### Summary of the Invention

We have discovered a class of polypeptides which react with an epitode specific for neoplastic cells. These polypeptides are not only excellent diagnostic tools, but also can induce apoptosis of the neoplastic cells to which they bind. This latter characteristic results in a treatment for neoplastic diseases that lacks the side-effects of many existing therapeutics.

The present invention features polypeptides, such as monoclonal antibodies that may be used in the diagnosis and treatment of a neoplasm. Accordingly, in the first aspect, the invention features a purified polypeptide that includes an antibody, or a functional fragment thereof, that induces apoptosis of a neoplastic cell, e.g., a colorectal adenocarcinoma, ovarian cancer, squamous cell lung carcinoma, lobular mammary carcinoma, stomach carcinoma, esophagial squamous cell carcinoma, pancreatic adenocarcinoma, lung adenocarcinoma, ductal mammary carcinoma, uterine adenocarcinoma, or prostate adenocarcinoma cell, to which it binds, but does not induce apoptosis of a non-neoplastic cell, where the antibody specifically binds to at least one of HT-29 (ATCC Accession No. HTB-38; DSMZ Accession No. ACC 299), CACO-2 (ATCC Accession No. HBT-37; DSMZ Accession No. ACC 169), COLO-320 (DSMZ Accession No. ACC 144), COLO-206F (DSMZ Accession No. ACC 21), or COLO-678 (DSMZ Accession No. 194) cells and not to non-neoplastic cells.

In the second aspect, the invention features a purified polypeptide that includes an antibody, or a functional fragment thereof, that induces apoptosis of a neoplastic cell to which it binds, but does not induce apoptosis of a non-neoplastic cell, where the antibody specifically binds to a colorectal adenocarcinoma, ovarian cancer, squamous cell lung carcinoma, lobular mammary carcinoma, stomach carcinoma, esophagial squamous cell carcinoma, pancreatic adenocarcinoma, lung adenocarcinoma, ductal mammary carcinoma, uterine adenocarcinoma, or prostate adenocarcinoma cell.

In the third aspect, the invention features a purified polypeptide including an antibody, or a functional fragment thereof, that inhibits cell proliferation when bound to a neoplastic cell, e.g., a colorectal adenocarcinoma, ovarian cancer, squamous cell lung carcinoma, lobular mammary carcinoma, stomach carcinoma, esophagial squamous cell carcinoma, pancreatic adenocarcinoma, lung adenocarcinoma, ductal mammary carcinoma, uterine adenocarcinoma, or prostate adenocarcinoma cell, but does not inhibit cell proliferation of a non-neoplastic cell, where the antibody specifically binds to at least one of HT-29 (ATCC Accession No. HTB-38; DSMZ Accession No. ACC 299), CACO-2 (ATCC Accession No. HBT-37; DSMZ Accession No. ACC 169), COLO-320 (DSMZ Accession No. ACC 144), COLO-206F (DSMZ Accession No. ACC 21), or COLO-678 (DSMZ Accession No. 194) cells and not to non-neoplastic cells.

In a fourth aspect, the invention features a purified polypeptide including an antibody, or a functional fragment thereof, that inhibits cell proliferation when bound to a neoplastic cell, but does not inhibit cell proliferation of a non-neoplastic cell, where the antibody specifically binds to a colorectal adenocarcinoma, ovarian cancer, squamous cell lung carcinoma, lobular mammary carcinoma, stomach carcinoma, esophagial squamous cell carcinoma, pancreatic adenocarcinoma, lung adenocarcinoma, ductal mammary carcinoma, uterine adenocarcinoma, or prostate adenocarcinoma cell and not to a non-neoplastic cell.

In desirable embodiments of the first four aspects of the invention, the purified polypeptide includes a sequence that is substantially identical to the amino acid sequence of SEQ ID NO:1 and/or SEQ ID NO:3. In addition, this amino acid sequence of the purified polypeptide may be identical to the sequence of SEQ ID NO:1 and/or SEQ ID NO:3. In other desirable embodiments, the purified polypeptide is a human antibody, e.g., a human monoclonal antibody. Furthermore, the functional fragment of the purified polypeptide may be a V_{L}, V_{H}, F_{V}, F_{C}, Fab, Fab', or F(ab')₂ fragment. Such a functional fragment may include a fragment that is substantially identical, or is identical, to the sequence of SEQ ID NO: 1 or SEQ ID NO:3. Desirably, the function of such a fragment is the ability to induce apoptosis of a neoplastic cell and not of a non-neoplastic cell or to inhibit the proliferation of a neoplastic cell and not of a non-neoplastic cell.

In the fifth aspect, the invention features a cell, such as a hybridoma, that produces a polypeptide, e.g., an antibody, that induces apoptosis of a neoplastic cell, e.g., a colorectal adenocarcinoma, ovarian cancer, squamous cell lung carcinoma, lobular mammary carcinoma, stomach carcinoma, esophagial squamous cell carcinoma, pancreatic adenocarcinoma, lung adenocarcinoma, ductal mammary carcinoma, uterine adenocarcinoma, or prostate adenocarcinoma cell, to which it binds, but does not induce apoptosis of a non-neoplastic cell, where the polypeptide specifically binds to at least one of HT-29 (ATCC Accession No. HTB-38; DSMZ Accession No. ACC 299), CACO-2 (ATCC Accession No. HBT-37; DSMZ Accession No. ACC 169), COLO-320 (DSMZ Accession No. ACC 144), COLO-206F (DSMZ Accession No. ACC 21), or COLO-678 (DSMZ Accession No. 194) cells and not to non-neoplastic cells.

In the sixth aspect, the invention features a cell, such as a hybridoma, that produces a polypeptide, e.g., an antibody, that inhibits cell proliferation in a neoplastic cell, e.g., a colorectal adenocarcinoma, ovarian cancer, squamous cell lung carcinoma, lobular mammary carcinoma, stomach carcinoma, esophagial squamous cell carcinoma, pancreatic adenocarcinoma, lung adenocarcinoma, ductal mammary carcinoma, uterine adenocarcinoma, or prostate adenocarcinoma cell, to which it binds, but not in a non-neoplastic cell, where the polypeptide specifically binds to at least one of HT-29 (ATCC Accession No. HTB-38; DSMZ Accession No. ACC 299), CACO-2 (ATCC Accession No. HBT-37; DSMZ Accession No. ACC 169), COLO-320 (DSMZ Accession No. ACC 144), COLO-206F (DSMZ Accession No. ACC 21), or COLO-678 (DSMZ Accession No. 194) cells and not to non-neoplastic cells.

The seventh aspect of the invention features a cell, such as a hybridoma, that produces a polypeptide, e.g., an antibody, that includes a sequence that is substantially identical to the amino acid sequence of SEQ ID NO:1, and in a desirable embodiment of this aspect, the sequence is identical to SEQ ID NO:1.

The eighth aspect of the invention features a cell, such as a hybridoma, that produces a polypeptide, e.g., an antibody, that includes a sequence that is substantially identical to the amino acid sequence of SEQ ID NO:3, and in a desirable embodiment of this aspect, the sequence is identical to SEQ ID NO:3.

The ninth aspect of the invention features a cell, such as a hybridoma, that produces a polypeptide, e.g., an antibody, that includes the amino acid sequence of SEQ ID NOS:1 and 3.

In a desirable embodiment of the fifth aspect, the invention features a method of generating the cell claimed in that aspect. This method involves the steps of (a) contacting lymphocytes with a heteromyeloma cell line under conditions that result in the fusion of a lymphocyte with a heteromyeloma cell, where this fusion results in a hybridoma (b) determining whether the hybridoma produces a polypeptide, e.g., a monoclonal antibody, that induces apoptosis of a neoplastic cell to which it binds, but does not induce apoptosis of a non-neoplastic cell, and (c) determining whether the hybridoma produces a polypeptide, e.g., a monoclonal antibody, that specifically binds to at least one of HT-29 (ATCC Accession No. HTB-38; DSMZ Accession No. ACC 299), CACO-2 (ATCC Accession No. HBT-37; DSMZ Accession No. ACC 169), COLO-320 (DSMZ Accession No. ACC 144), COLO-206F (DSMZ Accession No. ACC 21), or COLO-678 (DSMZ Accession No. 194) cells and not to non-neoplastic cells.

In a desirable embodiment of the sixth aspect, the invention features a method of generating the cell of that aspect. This method involves the steps of (a) contacting lymphocytes with a heteromyeloma cell line under conditions that result in the fusion of a lymphocyte with a heteromyeloma cell, where this fusion results in a hybridoma, (b) determining whether the hybridoma produces a polypeptide, e.g., a monoclonal antibody, that inhibits proliferation in a neoplastic cell to which it binds, but does not inhibit proliferation in a non-neoplastic cell, and (c) determining whether the hybridoma produces a polypeptide, e.g., a monoclonal antibody, that specifically binds to at least one of HT-29 (ATCC Accession No. HTB-38; DSMZ Accession No. ACC 299), CACO-2 (ATCC Accession No. HBT-37; DSMZ Accession No. ACC 169), COLO-320 (DSMZ Accession No. ACC 144), COLO-206F (DSMZ Accession No. ACC 21), or COLO-678 (DSMZ Accession No. 194) cells and not to non-neoplastic cells.

In a further desirable embodiment of the first four aspects, the invention features a method of diagnosing a neoplasm, such as a colorectal adenocarcinoma, ovarian cancer, squamous cell lung carcinoma, lobular mammary carcinoma, stomach carcinoma, esophagial squamous cell carcinoma, pancreatic adenocarcinoma, lung adenocarcinoma, ductal mammary carcinoma, uterine adenocarcinoma, or prostate adenocarcinoma, in a mammal, e.g., a human. This method involves the steps of (a) contacting a cell or tissue sample of the mammal with the purified polypeptide of these aspects of the invention, and (b) detecting whether the purified polypeptide binds to the cell or tissue sample, wherein binding of the purified polypeptide to the cell or tissue sample is indicative of the mammal having a neoplasm. In desirable embodiments of this method, the polypeptide, e.g., an antibody, is conjugated to a detectable agent, e.g., a radionuclide, a fluorescent marker, an enzyme, a cytotoxin, a cytokine, or a growth inhibitor, or the polypeptide is conjugated to a protein purification tag, e.g., a cleavable protein purification tag.

In additional desirable embodiments of the first four aspects, the invention features a method of treating a proliferative disorder, such as a colorectal adenocarcinoma, ovarian cancer, squamous cell lung carcinoma, lobular mammary carcinoma, stomach carcinoma, esophagial squamous cell carcinoma, pancreatic adenocarcinoma, lung adenocarcinoma, ductal mammary carcinoma, uterine adenocarcinoma, or prostate adenocarcinoma, in a mammal, e.g., a human. This method involves the step of contacting a cell or tissue sample with the purified polypeptide of these aspects, where binding of the purified polypeptide to the cell or tissue sample results in the induction of apoptosis of the cell or tissue sample. In desirable embodiments of this method, the polypeptide, e.g., an antibody, is conjugated to a detectable agent, e.g., a radionuclide, a fluorescent marker, an enzyme, a cytotoxin, a cytokine, or a growth inhibitor, or the polypeptide is conjugated to a protein purification tag, e.g., a cleavable protein purification tag.

The detectable agent may consist of a polypeptide that is specific for a neoplastic cell and a compound that is capable of killing the neoplastic cell, for example, by inducing apoptosis. The cell killing effect may be due entirely to the compound linked to the polypeptide, or is may be due to an additive or synergistic effect of the compound and the polypeptide contained in the detectable agent. Examples of compounds that can kill a cell include radionuclides, cytotoxins, and cytokines.

In other desirable embodiments of the first four aspects, the invention features a method of treating a proliferative disorder, such as a colorectal adenocarcinoma, ovarian cancer, squamous cell lung carcinoma, lobular mammary carcinoma, stomach carcinoma, esophagial squamous cell carcinoma, pancreatic adenocarcinoma, lung adenocarcinoma, ductal mammary carcinoma, uterine adenocarcinoma, or prostate adenocarcinoma, in a mammal, e.g., a human. This method involves the step of contacting a cell or tissue sample with the purified polypeptide of these aspects, where binding of the purified polypeptide to the cell or tissue sample results in a reduction in proliferation of the cell or of a cell in the tissue sample. In desirable embodiments of this method, the polypeptide, e.g., an antibody, is conjugated to a detectable agent, e.g., a radionuclide, a fluorescent marker, an enzyme, a cytotoxin, a cytokine, or a growth inhibitor, or the polypeptide is conjugated to a protein purification tag, e.g., a cleavable protein purification tag.

The detectable agent may consist of a polypeptide that is specific for a neoplastic cell and a compound, e.g., a growth inhibitor, which is capable of inhibiting the proliferation of the neoplastic cell. The proliferation inhibiting effect may be due entirely to the compound linked to the polypeptide, or is may be due to an additive or synergistic effect of the compound and the polypeptide contained in the detectable agent.

Additional desirable embodiments of the first four aspects of the invention include a medicament containing the purified polypeptide of any one of these aspects in a pharmaceutically acceptable carrier and a diagnostic agent containing the purified polypeptide of any one of these aspects of the invention.

### Definitions

By "detectable agent" is meant a compound that is linked to a diagnostic agent to facilitate detection. Such a "detectable agent" may be covalently or non-covalently linked to a diagnostic agent. In addition, the linkage may be direct or indirect. Examples of "detectable agents" include, protein purification tags, cytotoxins, enzymes, paramagnetic labels, enzyme substrates, co-factors, enzymatic inhibitors, dyes, radionuclides, chemiluminescent labels, fluorescent markers, growth inhibitors, cytokines, antibodies, and biotin.

By a "diagnostic agent" is meant a compound that may be used to detect a neoplastic cell by employing any one of the assays described herein as well as any other method that is standard in the art. A diagnostic agent may include, for example, an antibody which specifically binds to at least one of the following cells: HT-29 (ATCC Accession No. HTB-38; DSMZ Accession No. ACC 299), CACO-2 (ATCC Accession No. HBT-37; DSMZ Accession No. ACC 169), COLO-320 (DSMZ Accession No. ACC 144), COLO-206F (DSMZ Accession No. ACC 21), and COLO-678 (DSMZ Accession No. 194), but not to non-neoplastic cells. In addition, a "diagnostic agent" may inhibit cell proliferation, induce apoptosis, or both only when it is bound to a neoplastic cell, but not a non-neoplastic cell.

Examples of neoplastic cells that may be detected with such a "diagnostic agent" include colorectal adenocarcinoma, ovarian cancer, squamous cell lung carcinoma, lobular mammary carcinoma, stomach carcinoma, esophagial squamous cell carcinoma, pancreatic adenocarcinoma, lung adenocarcinoma, ductal mammary carcinoma, uterine adenocarcinoma, or prostate adenocarcinoma cells. Moreover, a "diagnostic agent" may include, for example, peptides, polypeptides, synthetic organic molecules, naturally-occurring organic molecules, nucleic acid molecules, and components thereof, as well as one or more detectable agent covalently or non-covalently linked to the diagnostic agent.

By a "functional fragment," as used herein in reference to polypeptide, e.g., an antibody, is meant, for example, a V_{L}, V_{H}, F_{V}, F_{C}, Fab, Fab', or F(ab')₂ fragment of an antibody (see, e.g., Huston et al., Cell Biophys. 22:189-224, 1993; and Harlow and Lane, Antibodies: A Laboratory Manual, Cold Spring Harbor Laboratory, 1988). Desirably, a "functional fragment" has an amino acid sequence that is substantially identical to a fragment, e.g., 5, 10, 15, 20, 15, 30, 50, 75, or 100 contiguous amino acids, of the amino acid sequence of SEQ ID NO:1 or 3. In more desirable embodiments, a "functional fragment" is identical to a fragment of the sequence of SEQ ID NO:1 or 3. Such a "functional fragment" may contain 5, 10, 15, 20, 15, 30, 50, 75, or 100 contiguous amino acids of SEQ ID NO:1 or 3, or may be the entire amino acid sequence of SEQ ID NO:1 or 3.

In addition, a "functional fragment" of a polypeptide has at least one biological activity of the full-length polypeptide. Examples of such a biological activity are the ability to bind an antigen, induce apoptosis, and/or inhibit cell proliferation. These biological activities may be determined, for example, using any one of the assays described herein.

By "high stringency hybridization conditions" is meant, for example, hybridization at approximately 42°C in about 50% formamide, 0.1 mg/ml sheared salmon sperm DNA, 1% SDS, 2X SSC, 10% Dextran sulfate, a first wash at approximately 65°C in about 2X SSC, 1% SDS, followed by a second wash at approximately 65°C in about 0.1X SSC. Alternatively, "high stringency hybridization conditions" may include hybridization at approximately 42°C in about 50% formamide, 0.1 mg/ml sheared salmon sperm DNA, 0.5% SDS, 5X SSPE, 1X Denhardt's, followed by two washes at room temperature in 2X SSC, 0.1% SDS, and two washes at between 55-60°C in 0.2X SSC, 0.1% SDS.

A "hybridoma," as used herein, is any cell that is artificially created by the fusion of a normal cell such as an activated lymphocyte with a neoplastic cell, e.g., a myeloma. The hybrid cell, which results from the fusion of at least two cells, may produce a monoclonal antibody or T cell product identical to those produced by the immunologically-competent parent. In addition, these cells, like the neoplastic parent, are immortal.

"Inhibiting cell proliferation," as used herein, refers to a reduction in the rate of cell division of a cell in comparison with the normal rate of cell division of that type of cell. Inhibition of cell proliferation may be assayed using a number of methods standard in the art, for example, the MTT cell proliferation assay described herein, BrdU incorporation, and ³H thymidine uptake. Such assays are described, for example, in Ausubel et al., Current Protocols in Molecular Biology, Wiley Interscience, New York, 2001; and Sambrook et al., Molecular Cloning: A Laboratory Manual, Cold Spring Harbor Laboratory, N.Y., 1989. Desirably, the inhibition of cell proliferation is 20%, 40%, 50%, or 75%. In desirable embodiments, the inhibition of cell proliferation is 80%, 90%, 95%, or even a complete inhibition of cell proliferation.

"Inducing apoptosis," as used herein, refers to the appearance of characteristics in a cell that are well defined in the art (see, e.g., Wyllie et al., Br. J. Cancer 80 Suppl. 1:34-37, 1999; Kerr et al., Br. J. Cancer 26:239-257, 1972). These characteristics include morphological characteristics, such as membrane blebbing, DNA condensation, as well as changes in F-actin content, mitochondrial mass, and membrane potential. The induction of apoptosis may be assayed using a number of methods standard in the art, for example, a cell death ELISA, TUNEL staining, DNA stains, e.g., Hoechst 33258, and staining with various vital dyes such as acridine orange, Mito Tracker Red^{®} staining (Molecular Probes, Eugene, OR), and Annexin V^{®} staining (Becton Dickinson, NJ). As used herein "inducing apoptosis" refers to an increase in the number of cells undergoing apoptosis when compared with a control cell population. For instance, the increase of apoptosis may be 10%, 20%, 40%, 50%, or 75%. In desirable embodiments, the induction of apoptosis results in an increase of apoptosis that is 2-fold, 3-fold, 10-fold, or even 100-fold over that seen in a control cell population.

A "neoplastic cell," as used herein, refers to a cell which is undergoing cell division, not undergoing apoptosis, or both, under inappropriate conditions. For example, a "neoplastic cell" may undergo cell division when a corresponding non-neoplastic cell does not undergo cell division, or, alternatively, a "neoplastic cell" may not respond to normal cell-cycle checkpoint controls.

A "proliferative disease," as used herein, refers to any disorder that results in the abnormal proliferation of a cell. Specific examples of proliferative diseases are various types of neoplasms, such as colorectal adenocarcinomas, ovarian cancer, squamous cell lung carcinomas, lobular mammary carcinomas, stomach carcinomas, esophagial squamous cell carcinomas, pancreatic adenocarcinomas, lung adenocarcinomas, ductal mammary carcinomas, uterine adenocarcinomas, or prostate adenocarcinomas. However, proliferative diseases may also be the result of the cell becoming infected with a transforming virus.

A "protein purification tag," as used herein, is a peptide, e.g., an epitope tag, that is covalently or non-covalently added to a protein to aid in the purification of the protein. Desirably such peptides bind with high affinity to an antibody or to another peptide such as biotin or avidin. Commercially available examples of epitope tags include His-tags, HA-tags, FLAG^{®}-tags, and c-Myc-tags. However, any epitope that is recognized by an antibody also may be used as a protein purification tag. See, for example, Ausubel et al., Current Protocols in Molecular Biology, Wiley Interscience, New York, 2001; and Sambrook et al., Molecular Cloning: A Laboratory Manual, Cold Spring Harbor Laboratory, N.Y., (1989). Protein purification tags may be cleaved from a protein, for example, by using an enzyme, e.g., thrombin, or a chemical, e.g., cyanogen bromide.

By "specifically recognize," as used herein in reference to a polypeptide, e.g., an antibody, is meant an increased affinity of a polypeptide for a particular protein, e.g., an antigen, relative to an equal amount of any other protein. For example, an antibody, e.g., the CM-1 human monoclonal antibody, that specifically binds to HT-29 (American Type Culture Collection ("ATCC") Accession No. HTB-38, German Collection of Microorganisms and Cell Cultures ("DSMZ") Accession No. ACC 299), CACO-2 (ATCC Accession No. HBT-37, DSMZ Accession No. ACC 169), COLO-320 (DSMZ Accession No. ACC 144), COLO-206F (DSMZ Accession No. ACC 21), or COLO-678 (DSMZ Accession No. 194) cells desirably has an affinity for its antigen that is least 2-fold, 5-fold, 10-fold, 30-fold, or 100-fold greater than for an equal amount of any other antigen, including related antigens. Binding of a polypeptide to another polypeptide may be determined as described herein, and by any number of standard methods in the art, e.g., Western analysis, ELISA, or co-immunoprecipitation.

By "substantially identical" is meant a polypeptide or nucleic acid exhibiting at least 75%, 80%, 85%, or 90% identity to a reference amino acid or nucleic acid sequence. In desirable embodiments, the polypeptide or nucleic acid sequence is at least 95%, 98%, 99%, or 100% identical to a reference amino acid or nucleic acid sequence. For polypeptides, the length of comparison sequences will generally be at least 5, 10, or 15 amino acids and desirably at least 20 or 25 contiguous amino acids. In more desirable embodiments, the length of comparison sequences is at least 30, 50, 75, 90, 95, or 100 contiguous amino acids, or even the full-length amino acid sequence. For nucleic acids, the length of comparison sequences will generally be at least 15, 30, or 45 contiguous nucleotides, and desirably at least 60 contiguous nucleotides. In more desirable embodiments, the length of comparison sequences is at least 75, 150, 225, 270, 285, or 300 contiguous nucleotides, or even the full-length nucleotide sequence.

Sequence identity may be measured using sequence analysis software on the default setting (e.g., Sequence Analysis Software Package of the Genetics Computer Group, University of Wisconsin Biotechnology Center, 1710 University Avenue, Madison, WI 53705). Such software may match similar sequences by assigning degrees of homology to various substitutions, deletions, and other modifications. Conservative substitutions typically include substitutions within the following groups: glycine, alanine, valine, isoleucine, leucine; aspartic acid, glutamic acid, asparagine, glutamine; serine, threonine; lysine, arginine; and phenylalanine, tyrosine.

Multiple sequences may also be aligned using the Clustal W(1.4) program (produced by Julie D. Thompson and Toby Gibson of the European Molecular Biology Laboratory, Germany and Desmond Higgins of European Bioinformatics Institute, Cambridge, UK) by setting the pairwise alignment mode to "slow," the pairwise alignment parameters to include an open gap penalty of 10.0 and an extend gap penalty of 0.1, as well as setting the similarity matrix to "blosum." In addition, the multiple alignment parameters may include an open gap penalty of 10.0, an extend gap penalty of 0.1, as well as setting the similarity matrix to "blosum," the delay divergent to 40%, and the gap distance to 8.

By "purified" is meant separated from other components that naturally accompany it. Typically, a factor is substantially pure when it is at least 50%, by weight, free from proteins, antibodies, and naturally-occurring organic molecules with which it is naturally associated. Desirably, the factor is at least 75%, more desirably, at least 90%, and most desirably, at least 99%, by weight, pure. A substantially pure factor may be obtained by chemical synthesis, separation of the factor from natural sources, or production of the factor in a recombinant host cell that does not naturally produce the factor. Proteins, vesicles, and organelles may be purified by one skilled in the art using standard techniques, such as those described by Ausubel et al. (Current Protocols in Molecular Biology, Wiley Interscience, New York, 2001). The factor is desirably at least 2, 5, or 10 times as pure as the starting material, as measured using polyacrylamide gel electrophoresis, column chromatography, optical density, HPLC analysis, or Western analysis (Ausubel et al., Current Protocols in Molecular Biology, Wiley Interscience, New York, 2001). Desirable methods of purification include immunoprecipitation, column chromatography such as immunoaffinity chromatography and nickel affinity columns, magnetic bead immunoaffinity purification, and panning with a plate-bound antibody.

Other features and advantages of the invention will be apparent from the following Detailed Description, the drawings, and the claims.

### Brief Description of the Drawings

Figure 1 is the amino acid (SEQ ID NO:1) and the nucleic acid (SEQ ID NO:2) sequence of the variable region of the heavy chain of the CM-1 human monoclonal antibody.
Figure 2 is the amino acid (SEQ ID NO:3) and the nucleic acid (SEQ ID NO:4) sequence of the variable region of the light chain of the CM-1 human monoclonal antibody.
Figure 3A-3C is a series of immunostains of a human ovarian adenocarcinoma. Figure 3A shows staining with the positive control antibody CAM 5.2. Figure 3B is a negative control, and Figure 3C shows staining with the CM-1 antibody.
Figure 4A-4C is a series of immunostains of a human ovarian adenocarcinoma. Figure 4A shows staining with the positive control antibody CAM 5.2. Figure 4B is a negative control, and Figure 4C shows staining with the CM-1 antibody.
Figure 5A-5C is a series of immunostains of a human invasive lobular carcinoma of the female breast. This series of pictures shows a typical satellite formation of tumor cells. Figure 5A shows staining with the positive control antibody CK 8. Figure 5B is a negative control, and Figure 5C shows staining with the CM-1 antibody.
Figure 6A-6C is a series of immunostains of a human invasive lobular carcinoma of the female breast. This series of pictures shows satellite formation surrounding ductal differentiation of the carcinoma. Figure 6A shows staining with the positive control antibody CK 8. Figure 6B is a negative control, and Figure 6C shows staining with the CM-1 antibody.
Figure 7A-7C is a series of immunostains of a human squamous cell carcinoma of the lung. Figure 7A shows staining with the positive control antibody CK 5/6. Figure 7B is a negative control, and Figure 7C shows staining with the CM-1 antibody.
Figure 8 is a graph of the results of a cell death enzyme-linked immunosorbent assay (ELISA) showing that the CM-1 monoclonal antibody induces apoptosis of CACO-2 cells.
Figure 9 is a series of graphs of the results of 3-(4,5-dimethylthiazol-2-yl) - 2,5-diphenyltetrazolium bromide (MTT) reduction assays for mitochondrial dehydrogenase activity showing that the CM-1 monoclonal antibody inhibits cell proliferation and decreases survival, or induces apoptosis of COLO-206F colon carcinoma cells after 24 hours of incubation (Figure 9A) and after 48 hours of incubation (Figure 9B).

### Detailed Description

The present invention features polypeptides, such as antibodies, and their use in the treatment and diagnosis of neoplasms. We have characterized a human monoclonal antibody (CM-1) that specifically recognizes a number of carcinomas, including colorectal adenocarcinomas, ovarian cancer, squamous cell lung carcinomas, lobular mammary carcinomas, stomach carcinomas, esophagial squamous cell carcinomas, pancreatic adenocarcinomas, lung adenocarcinomas, ductal mammary carcinomas, uterine adenocarcinomas, and prostate adenocarcinomas. Not only does the CM-1 monoclonal antibody recognize these neoplasms, but, upon binding to a cell, it can induce apoptosis of neoplastic cells, inhibit their proliferation, or even both. Thus, the CM-1 monoclonal antibody, and other antibodies, or fragments thereof, that are specific for the antigen recognized by CM-1, may be used in a variety of methods for diagnosing and treating a neoplasm.

The cell line that produces the human CM-1 monoclonal antibody was deposited on March 5, 2003 at the German Collection of Microorganisms and Cell Cultures ("DSMZ" - Deutsche Sammlung von Mikroorganismen und Zellkulturen GmbH, Mascheroder Weg 1b, 38124 Braunschweig, Germany) under the terms of the Budapest Treaty.

### Antibodies and Polypeptides

Antibodies play an essential role in maintaining the health of an individual. In particular, antibodies are present in serum and bind to and help eliminate diverse pathogens such as bacteria, viruses, and toxins. Antibodies consist of Y-shaped protein structures built from two heavy chains and two light chains. Each chain has a modular construction: each light chain consists of two domains, and each heavy chain has at least four domains. The antigen binding site is fashioned by one domain from the heavy chain (V_{H} domain) and one domain from the light chain (V_{L} domain). Indeed, small antigen binding fragments can be prepared by linking these two domains, either associated non-covalently, or covalently via disulphide bonds or a peptide linker. The antigen binding domains are more variable in amino acid sequence than the other domains of the antibody, and are therefore termed variable (V) domains, in contrast to the constant (C) domains. The constant domains of the antibody are responsible for triggering antibody effector mechanisms, such as complement lysis and cell-mediated killing.

Antibodies are made by B-lymphocytes in a process involving gene rearrangement. During the development of these cells, the genes encoding the variable domains are assembled from genetic elements. In the case of the V_{H} domains there are three elements, the un-rearranged V_{H} gene, D segment, and J_{H} segment. In the case of the V_{L} domains, there are two elements, the un-rearranged V_{L} (V Lambda or V Kappa) gene and the J_{L} (J Lambda or J Kappa) segment. Random combination of these gene segments and random combination of the rearranged V_{H} and V_{L} domains generate a large repertoire of antibodies, capable of binding to a large diversity of equally diverse antigens.

In general, the presently claimed polypeptide is any agent that binds to any one of HT-29, CACO-2, COLO-320, COLO-206F, or COLO-678, but does not bind to non-neoplastic cells. The polypeptide may be an antibody, such as a human monoclonal antibody (e.g., CM-1), or a functional fragment thereof. Overall, the polypeptide of the invention can exclusively bind to both neoplastic tissues and neoplastic cells, but not to non-neoplastic tissue or cells. The polypeptide also may induce apoptosis of a neoplastic cell to which it binds, but not in a non-neoplastic cell, or, alternatively, the polypeptide may inhibit proliferation of the neoplastic cell it binds to, but not in a non-neoplastic cell. Desirably, the polypeptide can simultaneously induce apoptosis and inhibit proliferation of neoplastic cells, but not of non-neoplastic cells. Such a polypeptide is, therefore, useful for the detection, monitoring, prevention, and treatment of cancers in mammals. Exemplary cancers amenable to the methods of the current invention include colorectal cancer, ovarian carcinoma, squamous cell lung carcinoma, small cell lung carcinoma, lobular and ductal mammary carcinomas, melanoma, breast cancer, lung cancer, such as lung adenocarcinomas, gastric cancer, pancreatic cancer, such as pancreatic adenocarcinomas, glioma, sarcomas, gastrointestinal cancer, brain tumor, esophageal cancer, such as esophagial squamous cell carcinomas, stomach cancer, osteosarcoma , fibrosarcomas, urinary bladder cancer, prostate cancer, such as prostate adenocarcinomas, renal cancer, ovarian cancer, testicular cancer, endometrial cancer, cervical cancer, uterine adenocarcinomas, Hodgkin's disease, lymphomas, and leukemias. The polypeptide is particularly useful for the detection of a lobular mammary carcinoma, ovarian carcinoma, lung squamous cell carcinoma, stomach carcinoma, esophagial squamous cell carcinoma, pancreatic adenocarcinoma, lung andenocarcinoma, ductal mammary carcinoma, uterine adenocarcinoma, and prostate adenocarcinoma.

### Production

The polypeptide according to the claimed invention can be produced by any method known in the art for small scale, large scale, or commercial production of polypeptides. For example, monoclonal antibodies, such as CM-1, may be produced by hybridoma cell lines. Such cell lines are typically generated by the fusion of spleen and lymph node lymphocytes derived from patients having a neoplasm, such as colon carcinoma, with a heteromyeloma cell line. Exemplary heteromyeloma cell lines include, for example, HAB-1 (Vollmers et al, Cancer 74:1525-1532, 1994), CB-F7 (Delvig et al., Hum. Antibodies Hybridomas 6:42-46, 1995), K6H6B5 (Delvig et al., Hum. Antibodies Hybridomas 6:42-46, 1995), H7NS.934 (Delvig et al., Hum. Antibodies Hybridomas 6:42-46, 1995), SHM-D33 (Bron et al., Proc. Natl. Acad. Sci. USA 81:3214-3217, 1984), and B6B11 (Borisova et al., Vopr. Virusol. 44:172-174, 1999). The ability to generate human monoclonal antibodies from lymphocytes of cancer patients allows the isolation of antibodies that are generated by an immune response in the cancer patient to the tumor.

Typically, portions of the lymph nodes and spleen are surgically removed from a patient having cancer, such as colon carcinoma. Lymphocytes may be prepared as cell suspensions by mechanical means and subsequently fused at, for example, a 1:2 or 1:3 ratio with a heteromyeloma cell line under conditions that result in cell fusion. For instance, the heteromyeloma cell line HAB-1, which is generated by the fusion of a human lymphocyte with the mouse myeloma NS-0, may be used for this purpose. A proportion of lymphocytes isolated from the cancer patient may also be maintained in culture. These cells serve as a source of human autologous cells useful for the initial antibody screening described below.

Following the fusion of the lymphocytes derived from the cancer patient with the heteromyeloma cell line, an antibody producing hybridoma or trioma is generated. Once constructed, hybridomas are generally stable in growth and antibody production in standard and mass cultures (flasks, miniPerm, fermenters, etc.) for several months. Levels of antibody production typically range between 0.01-0.1 mg/mL in flasks and between 0.1-0.5 mg/mL in miniPerm. Cell fusion may be achieved by any method known in the art, and includes, for example, the use of 40% polyethylene glycol. Hybridomas may be cultured in media containing HAT (Hypovanthin-aminopterin-thymidin) and after four weeks, supernatants may be screened for antibody production using an ELISA assay. Positive clones may then be tested in attachment inhibition and binding assays using autologous cell lines as prepared above. Positive clones further may be tested using immunoperoxidase staining of tumor and normal tissues. Thus, clones may be selected on the basis of their reactivity with autologous and allogeneic neoplastic cells. The antibody may be purified from mass cultures with use of cation-exchange chromatography followed by gel filtration as described, for example, by Vollmers et al., (Oncology Reports 5:35-40, 1998). Following the production of antibodies, additional functional and immunohistochemical tests of the antibodies produced by the trioma may be performed. For example, the antibodies produced by the hybridoma can be tested for their ability to induce apoptosis, inhibit cellular proliferation, or both, relative to untreated control cells. The antibodies can also be tested for their ability to specifically bind the neoplastic cell lines HT-29, CACo-2, COLO-320, COLO-206F, or COLO-678, relative to non-neoplastic cells.

Alternatively, the polypeptide, including an antibody, or a fragment thereof, may be produced by the expression of the polypeptide or antibody in a host cell such as *E. coli* or yeast, e.g., *S. cerevisiae.* For example, an antibody of the invention may be identified as follows. A nucleic acid sequence encoding an antibody, or a fragment thereof, may be inserted into filamentous bacteriophage to generate libraries of approximately 10⁷ or more antibodies. Each phage expresses an antibody on its surface that is encoded by the nucleic acid it contains. Antibodies of the invention may thus be screened and detected by functional and histochemical assays as described herein, and such genes may be subsequently selected and expressed in *E.coli.* This system is described, for example, in U.S. Patent No. 5,876,691.

Antibodies, or functional fragments thereof, may also be generated using, for example, direct synthesis using recombinant methods. These methods are standard in the art. For example, a nucleic acid sequence may be amplified using the polymerase chain reaction (PCR). The PCR technique is known in the art and is described, for example in U.S. Patent No. 4,683,195. Using standard methods, and as described herein, the sequence of a monoclonal antibody expressed by a hybridoma may be obtained and functional fragments of the antibody may be amplified. For example, whole RNA may be isolated from a hybridoma expressing a tumor-specific monoclonal antibody. cDNA may then be generated from the RNA using reverse transcriptase and the cDNAs which contain the functional fragments of the variable regions of the heavy and light chains may be amplified using PCR. The PCR products may then be purified and cloned into expression vectors. Many standard vectors are available and the selection of the appropriate vector will depend on, for example, the size of the DNA inserted into the vector and the host cell to be transformed with the vector.

### Isolation of Amino Acid Variants of a Polypeptide

Amino acid sequence variants of a polypeptide, such as an antibody, e.g., a CM-1 antibody, can be prepared by introducing appropriate nucleotide changes into the DNA encoding the antibody, or by *in vitro* synthesis of the desired polypeptide. Such variants include, for example, deletions from, or insertions or substitutions of, residues within the amino acid sequence of the CM-1 antibody. Any combination of deletion, insertion, and substitution can be made to arrive at the final construct, provided that the final construct possesses the desired characteristics, e.g., the ability to induce apoptosis of a neoplastic cell, but not a non-neoplastic cell or the ability to inhibit the proliferation of a neoplastic cell, but not a non-neoplastic cell. The amino acid changes also may alter post-translational processes of an antibody, such as changing the number or position of glycosylation sites, altering the membrane anchoring characteristics, or modifying its susceptibility to proteolytic cleavage.

In designing amino acid sequence variants of a polypeptide, such as an antibody, the location of the mutation site and the nature of the mutation will depend on characteristic(s) to be modified. The sites for mutation can be modified individually or in series, e.g., by (1) substituting first with conservative amino acid choices and then with more radical selections depending upon the results achieved, or (2) deleting the target residue.

A useful method for identification of specific residues or regions for mutagenesis in a polypeptide is called "alanine scanning mutagenesis" and is described, for example, by Cunningham and Wells (Science 244: 081-1085, 1989). Here, a residue or group of target residues are identified (e.g., charged residues such as arg, asp, his, lys, and glu) and replaced by a neutral or negatively charged amino acid (most desirably alanine or polyalanine) to affect the interaction of the amino acids with the surrounding aqueous environment in or outside the cell. Those domains demonstrating functional sensitivity to the substitutions then are refined by introducing further or other variants at or for the sites of substitution. Thus, while the site for introducing an amino acid sequence variation is predetermined, the nature of the mutation need not be predetermined. For instance, to optimize the performance of a mutation at a given site, alanine scanning or random mutagenesis may be conducted at the target codon or region and the expressed variants are screened for, e.g., the ability to induce apoptosis of a neoplastic cell and not a non-neoplastic cell, or to inhibit the proliferation of a neoplastic cell and not a non-neoplastic cell.

The sites of greatest interest for substitutional mutagenesis include sites identified as affecting the biological activity of a polypeptide. These sites, especially those falling within a sequence of at least three other identically conserved sites, may be substituted in a relatively conservative manner. For instance, ala may be substituted with val, leu, or ile; arg may be substituted with lys, gln, or asn; asn may be substituted with gln, his, lys, or arg; asp may be substituted with glu; cys may be substituted with ser; gln may be substituted with asn; glu may be substituted with asp; gly may be substituted with pro; his may be substituted with asn, gln, lys, or arg; ile may be substituted with leu, val, met, ala, or phe; leu may be substituted with ile, val, met, ala, or phe; lys may be substituted with arg, gln, or asn; met may be substituted with leu, phe, or ile; phe may be substituted with leu, val, ile, or ala; pro may be substituted with gly; ser may be substituted with thr; thr may be substituted with ser; trp may be substituted with tyr; tyr may be substituted with trp, phe, thr, or ser; and val may be substituted with ile, leu, met, or phe.

### Conjugation of the Antibody with a Detectable Agent

If desired, the claimed polypeptide such as an antibody (e.g., monoclonal antibody, such as CM-1), or a fragment thereof, may be linked to a detectable agent to facilitate the purification of the polypeptide as well as the diagnosis, monitoring, or treatment of cancer in a mammal in need thereof. The selection of suitable detectable agent will depend on the intended use of the polypeptide and will be apparent to those of ordinary skill in the art. Detectable agents according to the claimed invention include, for example, protein purification tags, cytotoxins, enzymes, paramagnetic labels, enzyme substrates, co-factors, enzyme inhibitors, dyes, radionuclides, chemiluminescent labels, fluorescent markers, growth inhibitors, and biotin.

A protein purification tag may be conjugated to the polypeptide of the invention, to facilitate isolation of the polypeptide. Examples of tags that can be used include His-tags, HA-tags, FLAG^{®}-tags, and c-Myc tags. An enzymatic or chemical cleavage site may be engineered between the polypeptide and the tag moiety so that the tag can be removed following purification. Suitable toxins include diphtheria toxin, Pseudomonas exotoxin A, ricin, and cholera toxin. Examples of suitable enzyme labels include malate hydrogenase, staphylococcal nuclease, delta-5-steroid isomerase, alcohol dehydrogenase, alpha-glycerol phosphate dehydrogenase, triose phosphate isomerase, peroxidase, alkaline phosphatase, asparaginase, glucose oxidase, beta-galactosidase, ribonuclease, urease, catalase, glucose-6-phosphate dehydrogenase, glucoamylase, and acetylcholinesterase. Examples of suitable radioisotopic labels include ³H, ¹²⁵I, ¹³¹I, ³²P, ³⁵S, and ¹⁴C. Preferably, the radioisotope will emit in the 10-5,000 kev range, more preferably 100-500 kev. Paramagnetic isotopes may also be conjugated to the polypeptide and used *in vivo* for the diagnosis and treatment of cancer. The use of such conjugated antibodies may be for *in vivo* nuclear magnetic resonance imaging. Such a method has previously been described (see, for example, Schaefer et al., JACC 14:472-480, 1989; Shreve et al., Magn. Reson. Med. 3:336-340, 1986; Wolf, Physiol. Chem. Phys. Med. NMR 16:93-95, 1984; Wesbey et al., Physiol. Chem. Phys. Med. NMR 16:145-155, 1984; and Runge et al., Invest. Radiol. 19:408-415, 1984). Alternatively, the radiolabeled antibody may also be used in radioimmunoguided surgery (RIGS), which involves the surgical removal of any tissue the labeled antibody binds to. Thus, the labeled antibody guides the surgeon towards neoplastic tissue by distinguishing it from non-neoplastic tissue. Radiolabels useful for tumor imaging are preferably short-lived radioisotopes. Various radioactive metals with half-lives ranging from 1 hour to 11.4 days are available for conjugation to antibodies, such as scandium-47 (3.4 days), gallium-67 (2.8 days), gallium-68 (68 minutes), technetium-99m (6 hours), indium-111 (3.2 days), and radium-223 (11.4 days), of which gallium-67, technetium-99m, and indium-111 are preferable for gamma camera imaging, gallium-68 is preferable for positron emission tomography, and scandium-47 and radium-223 (and other alpha-emitting radionuclides) are preferable for tumor therapy.

Examples of suitable fluorescent markers include fluorescein, isothiocyalate, rhodamine, phycoerythrin, phycocyanin, allophycocyanin, ophthaldehyde, and fluorescamine. Examples of chemiluminescent markers include a luminal label, isoluminal label, aromatic acridinium ester label, imidazole label, acridinium salt label, oxalate ester label, luciferin label, luciferase label, and aequorin label. Those of ordinary skill in the art would know of other suitable labels, which may be employed in accordance with the present invention. Conjugation of these detectable agents to the claimed polypeptides such as monoclonal antibodies, or fragments thereof, can be accomplished using standard techniques commonly known in the art. Typical antibody conjugation techniques are described by Kennedy et al. (Clin. Chim. Acta 70, 1-31, 1976) and Schurs et al. (Clin. Chim. Acta 81, 1-40, 1977) and include, for example, the glutaraldehyde method, the periodate method, the dimaleimide method, the m-maleimidobenzyl-N-hydroxy-succinimide ester method. Antibodies may be radiolabeled by any of several techniques known to the art, described, for example, in U.S. patent No. 4,444,744. All of these methods are incorporated by reference herein.

In all aspects of the present invention, it is understood that mixtures of different or the same labeled polypeptides specific to different antigens or different epitopes of the same antigen associated with the same or different tumor or tumor cell types may be used. Such a combination may enhance detection, localization and/or therapy in certain cases, and can also increase the range of a broad screen for more than one neoplasm or type of neoplasm.

### Polypeptides Conjugated to Anti-Tumor Agents

Although the polypeptide of the invention may induce apoptosis of neoplastic cells, inhibit cellular proliferation of neoplastic cells, or both, the polypeptide may in addition be conjugated to an agent that kills neoplastic cells or that inhibits their proliferation. The targeting ability of the polypeptide, such as an antibody or fragment thereof, results in the delivery to deliver of the cytotoxic or anti-proliferative agent to the tumor to enhance the destruction of the tumor. The polypeptide therefore may be used for the treatment and prevention of cancer in a mammal, such as a human patient. The cytotoxic agent linked to the polypeptide may be any agent that destroys or damages a tumor cell or tumor to which the polypeptide has bound. Examples of such agents include chemotherapeutic agents or radioisotopes, enzymes which activates a pro-drug, or a cytokine.

Suitable chemotherapeutic agents are known to those skilled in the art and include, for example, taxol, mithramycin, deoxyco-formycin, mitomycin-C, L-asparaginase, interferons (especially IFN-alpha), etoposide, teniposide, anthracyclines (e.g., daunomycin and doxorubicin), methotrexate, vindesine, neocarzinostatin, cis-platinum, chlorambucil, cytosine arabinoside, 5-fluorouridine, melphalan, ricin, and calicheamicin. The chemotherapeutic agents may be conjugated to the antibody using conventional methods known in the art.

Suitable radioisotopes for use as cytotoxic agents are also known to those skilled in the art and include, for example, ¹³¹I, or an astatine such as ²¹¹At. These isotopes may be attached to the polypeptide, either covalently or non-covalently, using conventional techniques known in the art.

Alternatively, the cytotoxic agent may also be an enzyme, which activates a pro-drug. This allows the conversion of an inactive pro-drug to its active, cytotoxic form at the tumor site and is called "antibody-directed enzyme pro-drug therapy" (ADEPT). Thus, the polypeptide-enzyme conjugate may be administered to the patient and allowed to localize in the region of the tumor to be treated. The pro-drug is then administered to the patient such that conversion to the cytotoxic drug is localized in the region of the tumor to be treated under the influence of the localized enzyme. An exemplary enzyme is bacterial carboxypeptidase G2 (CPG2) the use of which is described in, for example, WO 88/07378. The polypeptide-enzyme conjugate may, if desired, be modified in accordance with the teaching of WO 89/00427, such as to accelerate its clearance from areas of the body that are not in the vicinity of a neoplasm. The polypeptide-enzyme conjugate may also be used in accordance with WO 89/00427, for example, by providing an additional component, which inactivates the enzyme in areas of the body that are not in the vicinity of the tumor.

As another alternative, the cytotoxic agent conjugated to the claimed polypeptide may also be a cytokine such as interleukin-2 (IL-2), interleukin-4 (IL-4), or tumor necrosis factor alpha (TNF-alpha). The polypeptide targets the cytokine to the tumor so that the cytokine mediates damage to or destruction of the tumor without affecting other tissues. The cytokine may be fused to the polypeptide at the DNA level using conventional recombinant DNA techniques.

In addition, any inhibitor of cell proliferation. e.g., genistein, tamoxifen, or cyclophosphamide, may be conjugated with a polypeptide of the invention.

### Dosage

With respect to the therapeutic methods of the invention, it is not intended that the administration of the claimed polypeptide to a patient be limited to a particular mode of administration, dosage, or frequency of dosing; the present invention contemplates all modes of administration, including intramuscular, intravenous, intraperitoneal, intravesicular, intraarticular, intralesional, subcutaneous, or any other route sufficient to provide a dose adequate to decrease the number of neoplastic cells by inducing apoptosis of neoplastic cells, by inhibiting proliferation of tumor cells, or both. The compound(s) may be administered to the patient in a single dose or in multiple doses. When multiple doses are administered, the doses may be separated from one another by, for example, one day, two days, one week, two weeks, or one month. For example, the polypeptide (e.g., a monoclonal antibody, such as CM-1) may be administered once a week for, e.g., 2, 3, 4, 5, 6, 7, 8, 10, 15, 20, or more weeks. It is to be understood that, for any particular subject, specific dosage regimes should be adjusted over time according to the individual need and the professional judgment of the person administering or supervising the administration of the compositions. The precise dose will vary dependent on the polypeptide used, the density, on the tumor surface, of the ligand to which the polypeptide binds, and the rate of clearance of the polypeptide. For example, the dosage of the CM-1 antibody can be increased if the lower dose does not provide sufficient anti-neoplastic activity. Conversely, the dosage of the CM-1 antibody can be decreased if the neoplasm is cleared from the patient.

While the attending physician ultimately will decide the appropriate amount and dosage regimen, a therapeutically effective amount of the claimed polypeptide, such as a monoclonal antibody or a fragment thereof, may be, for example, in the range of about 0.1 mg to 50 mg/kg body weight/day or 0.70 mg to 350 mg/kg body weight/week. Desirably a therapeutically effective amount is in the range of about 0.50 mg to 20.0 mg/kg, and more desirably in the range of about 0.50 mg to 15.0 mg/kg for example, about 0.2, 0.3, 0.5, 1.0, 1.5, 2.0, 2.5, 3.0, 3.5, 4.0, 4.5, 5.0, 5.5, 6.0, 7.0, 8.0, 8.5, 9.0, 10.0, 11.0, 12.0, 13.0, 14.0, or 15.0 mg/kg body weight administered daily, every other day, or twice a week.

For example, a suitable dose is an amount of the polypeptide that, when administered as described above, is capable of inducing apoptosis, and is at least 20% above the basal (i.e., untreated) level. In general, an appropriate dosage and treatment regimen provides the active compound(s) in an amount sufficient to provide therapeutic and/or prophylactic benefit. Such a response can be monitored by establishing an improved clinical outcome (e.g., more frequent remissions, complete or partial, or longer disease-free survival) in treated patients as compared to non-treated patients. According to this invention, the administration of the polypeptide can induce neoplastic cell apoptosis by at least 20%, 40%, 50%, or 75% above that of an untreated control as measured by any standard assay known in the art. More preferably, apoptosis is induced by 80%, 90%, 95%, or even 100% above that of an untreated control. Alternatively, the administration of the polypeptide can inhibit neoplastic cell proliferation by at least 20%, 40%, 50%, or 75% below that of an untreated control as measured by any standard assay known in the art. More desirably, proliferation is inhibited by 80%, 90%, 95%, or even 100% below that of an untreated control. Most desirably, the polypeptide can simultaneously inhibit proliferation and induce apoptosis of neoplastic cells relative to untreated control cells. Such responses can be monitored by any standard technique known in the art. In general, for pharmaceutical compositions, the amount of antibody present in a dose ranges from about 25 µg to 5 mg per kg of host. Suitable dose sizes will vary with the size of the patient, but will typically range from about 0.1 mL to about 5 mL.

### Formulation of Pharmaceutical Compositions

The claimed polypeptide may be administered by any suitable means that results in a concentration having anti-neoplastic properties upon reaching the target region. The polypeptide may be contained in any appropriate amount in any suitable carrier substance, and is generally present in an amount of 1-95% by weight of the total weight of the composition. The composition may be provided in a dosage form that is suitable for parenteral (e.g., subcutaneous, intravenous, intramuscular, or intraperitoneal) administration route. The pharmaceutical compositions may be formulated according to conventional pharmaceutical practice (see, e.g., Remington: The Science and Practice of Pharmacy (20th ed.), ed. A.R. Gennaro, Lippincott Williams & Wilkins, 2000 and Encyclopedia of Pharmaceutical Technology, eds. J. Swarbrick and J. C. Boylan, 1988-1999, Marcel Dekker, New York).

The pharmaceutical composition may be administered parenterally by injection, infusion or implantation (subcutaneous, intravenous, intramuscular, intraperitoneal, or the like) in dosage forms, formulations, or via suitable delivery devices or implants containing conventional, non-toxic pharmaceutically acceptable carriers and adjuvants. If the neoplastic cells are in direct contact with the blood (e.g., leukemias), or if the tumor is only accessible by the bloodstream then the intravenous (I.V.) route may be used. In cases in which tumors grow in confined spaces such as the pleural cavity or the peritoneal cavity, the polypeptide may be directly administered into the cavity rather than into the blood stream. The formulation and preparation of such compositions are well known to those skilled in the art of pharmaceutical formulation. Formulations can be found in Remington: The Science and Practice of Pharmacy, *supra.*

### Diagnosis and Monitoring Cancer Progression

As discussed above, the present invention is directed to a method for detecting or diagnosing a neoplasm in a mammal, preferably a human patient. Typically, any neoplasm in which administration of the claimed polypeptide causes an induction in apoptosis or a reduction in proliferation are amenable to the methods of this invention.

The claimed polypeptides are particularly useful since they are specific to neoplasms or neoplastic cells, but not normal cells or tissue. Accordingly, this polypeptide can bind to neoplastic cells within the tumor, but not the normal surrounding tissue, thus allowing the detection, the treatment, or both, of a neoplasm in a mammal. For instance, one may use a polypeptide of the invention to determine is a biopsy removed the entire tumor by verifying that no cells bound by the polypeptide remain in the patient or, by verifying that tumor removed from the patient is entirely surrounded by cells that are not bound by the polypeptide.

It is understood that to improve the sensitivity of detection, multiple neoplastic markers may be assayed within a given sample or individual. Thus, polypeptides such as antibodies or functional fragments specific for different antigens may be combined within a single assay, or in multiple assays. Further, multiple primers or probes specific to neoplasms may be used concurrently. The selection of markers may be based on routine experiments to determine combinations that results in optimal sensitivity.

### In Vitro Detection of a Neoplasm

In general, the diagnosis of a neoplasm in a mammal involves obtaining a biological sample from the mammal (e.g., human patient), contacting such sample with the polypeptide of the invention (e.g., a monoclonal antibody, such as CM-1), detecting in the sample the level of reactivity or binding of the polypeptide to neoplastic cells relative to a control sample, which corresponds to non-neoplastic cells derived from healthy tissue from the mammal in which the cancer is being diagnosed or from another patient known not to have neoplasm. Thus, the methods of this invention are particularly useful for the detection of early stage tumors or metastases, which are otherwise undetectable. Accordingly, in addition to diagnosing a neoplasm in a patient, the methods of this invention may also be used to monitor progression of a neoplasm in a mammal. The polypeptides described herein therefore may be used as markers for the progression of a neoplasm. For this purpose, the assays described below, which are used for the diagnosis of a neoplasm, may be performed over time, and the change in the level of reactive polypeptide(s) evaluated. For example, the assays may be performed every 24-72 hours for a period of 6 months to 1 year, and thereafter performed as needed. In general, a neoplasm is progressing in those patients in whom the level of bound polypeptide detected increases over time. In contrast, the neoplasm is not progressing when the level of bound polypeptide either remains constant or decreases with time. Alternatively, as is noted above, the polypeptide of the invention may also be used to determine the presence of tumor cells in the mammal following tumor resection by surgical intervention to determine whether the tumor has been completely removed from the mammal.

Desirably, the polypeptide is linked to a detectable agent, which facilitates detection, or measurement of polypeptide reactivity. The biological sample is any biological material, which may contain neoplastic cells and include, for example, blood, saliva, tissue, serum, mucus, sputum, urine, or tears. The biological sample may also be a tissue section, which may be fixed tissue, fresh tissue, or frozen tissues. A neoplasm is detected or diagnosed in the mammal from which the sample was obtained if there is an increase in the level of reactivity of the antibody with the biological sample over the control sample. Such increase is at least 10%, 20%, 30%, 40%, 50%, or more than 50% over control levels. The level of binding or reactivity can be determined by any method known in the art and is described in further detail below.

### In Vitro Diagnostic Assays

The diagnosis of neoplasms using the claimed polypeptide may be performed by any method known to those of ordinary skill in the art for using a binding agent to detect polypeptide markers in a sample. See, e.g., Harlow and Lane, Antibodies: A Laboratory Manual, Cold Spring Harbor Laboratory, 1988. For example, the polypeptide may be used for enzyme-linked immunosorbent assay (ELISA), Western blotting or *in situ* detection of tumor cells in a tissue sample. For example, the ELISA assay typically involves the use of the polypeptide, such as an antibody, immobilized on a solid support to bind to the tumor cells in the biological sample. The bound tumor cell may then be detected using a detection reagent that contains a reporter group and that specifically binds to the antibody/tumor cell complex. Such detection reagents include, for example, any binding agent that specifically binds to the antibody, such as an anti-immunoglobulin, protein G, protein A, or a lectin. Alternatively, a competitive assay may be utilized, in which the polypeptide is an antibody and in which the antigens, to which the antibody is specific to is labeled with a reporter group and allowed to bind to the immobilized antibody after incubation of the antibody with the biological sample. The extent to which components of the sample inhibit the binding of the labeled antigens to the antibody is indicative of the reactivity of the sample with the immobilized antibody. Diagnosis of a neoplasm in a patient may also be determined by a two-antibody sandwich assay. This assay may be performed by first contacting an antibody that has been immobilized on a solid support, commonly the well of a microtiter plate, with the sample, such that polypeptides within the sample are allowed to bind to the immobilized antibody. Unbound sample is then removed from the immobilized polypeptide-antibody complexes and a detection reagent (preferably a second antibody capable of binding to a different site on the polypeptide) containing a reporter group is added. The amount of detection reagent that remains bound to the solid support is then determined using a method appropriate for the specific reporter group. For example, to determine the presence or absence of a neoplasm, such as colorectal adenocarcinoma, the signal detected from the reporter group that remains bound to the solid support is generally compared to a signal that corresponds to a predetermined cut-off value. The cut-off value for the detection of a neoplasm is the average mean signal obtained when the antibody is incubated with samples from patients without a neoplasm.

The method employed for detecting the reporter group depends upon the nature of the reporter group. For radioactive groups, scintillation counting or autoradiographic methods may be used. Spectroscopic methods may be used to detect dyes, luminescent groups and fluorescent groups. Biotin may be detected using avidin, coupled to a different reporter group (commonly a radioactive or fluorescent group or an enzyme). Enzyme reporter groups may generally be detected by the addition of substrate (generally for a specific period of time), followed by spectroscopic or other analysis of the reaction products.

The polypeptide of the invention may also be employed histologically for *in situ* detection or quantitative determination of tumor cells, for example, by immunofluorescence or immunoelectron microscopy. *In situ* detection or determination may be accomplished by removing a tissue specimen from a patient and allowing a labeled antibody to bind to any tumor cell in the specimen. Using such a procedure not only allows the detection of neoplastic cells in a sample, but also allows for the determination of their spatial distribution. As another example, the biological sample can be a smear of biological material containing neoplastic cells on a slide, and the detection of neoplastic cells in the biological material is achieved by examining the smear with a microscope or by flurocytometry.

### In Vivo detection of a Neoplasm

Alternatively, the antibody of the invention may also be used *in vivo* for detecting and localizing a neoplasm. Such a method may involve injecting a mammal, desirably a human subject, parenterally with the polypeptide of the invention, such as CM-1, which has been labeled with a detectable agent, and is described, for instance, in U.S. Patent No. 4,444,744. For example, the polypeptide can be radiolabeled with a pharmacologically inert radioisotope and administered to the patient. The activity of the radioisotope can be detected in the mammal using a photoscanning device, and an increase in activity relative to a control reflects the detection and localization of a neoplasm.

### Treatment

In addition to the diagnosis and monitoring of neoplasms in mammals, the present invention also features methods for treating neoplasms in a mammal, desirably a human patient. The method generally involves the administration of a biologically effective amount of the polypeptide of the invention to the patient. The polypeptide is typically administered to the mammal by means of injection using any routes of administration such as by intrathecal, subcutaneous, submucosal, or intracavitary injection as well as for intravenous or intraarterial injection. Thus, the polypeptide may be injected systemically, for example, by the intravenous injection of the polypeptide such as the CM-1 antibody into the patient's bloodstream or alternatively, the polypeptide can be directly injected at the site of the neoplasm or at a location in proximity to the neoplastic cells.

In general and as discussed above, binding of the polypeptide of the invention to neoplastic cells results in an induction in apoptosis, a reduction in cellular proliferation, or both relative to the control sample. Alternatively, the antibodies may also activate the complement pathway, which ultimately causes holes to be punctured on the cellular membrane, resulting in cell death.

If desired, the polypeptides may also be conjugated to drugs or toxins as described above. Once attached to the cell surface, the conjugate may be engulfed into the cell cytoplasm where cell enzymes cleave, and, thus, activate or free the drugs or toxins from the conjugate. Once released, the drugs or toxins damage the cell and irreversibly induce cell death. With respect to radiolabeled antibodies, binding to neoplastic cells and the resulting emission of radiation, at a short distance from the cell DNA, produces damage to the latter thus inducing cell death in the next replication round. For example, after a neoplasm has been detected and localized in a subject, a higher dose of labeled antibody, generally from 25 to 250 mCi for ¹³¹I, and preferably from 50 nCi to 150 mCi per dose, based on a 70 kg patient weight, is injected. Injection may be intravenous, intraarterial, intralymphatic, intrathecal, or intracavitary, and may be repeated more than once. It may be advantageous for some therapies to administer multiple, divided doses of radiolabeled polypeptides or polypeptide mixtures, e.g., in the range of 20-120 mCi (70 kg patient), thus providing higher cell-killing doses to the neoplasm without usually effecting a proportional increase in radiation of normal tissues

Therapy using labeled polypeptides is advantageously used as a primary therapeutic treatment, but may also be used in combination with other anti-neoplastic therapies, e.g., radiation and chemotherapy, and as an adjunct to surgery. The administration of such conjugated polypeptides is particularly useful in the case where small metastases cannot be surgically removed.

### Combination of a Polypeptide with other Anti-Neoplastic Therapies

Chemotherapeutic agents and/or radiation and/or surgical removal of the neoplasm can optionally be combined with any of the methods of the present invention. Classes of compounds that can be used as the chemotherapeutic agent include: alkylating agents, antimetabolites, natural products and their derivatives, hormones and steroids (including synthetic analogs), and synthetics. Examples of alkylating agents (e.g., nitrogen mustards, ethylenimine derivatives, alkyl sulfonates, nitrosoureas and triazenes) include Uracil mustard, Chlormethine, Cyclophosphamide (Cytoxan^{RTM}), Ifosfamide, Melphalan, Chlorambucil, Pipobroman, Triethylene-melamine, Triethylenethiophosphoramine, Busulfan, Carmustine, Lomustine, Streptozocin, Dacarbazine, and Temozolomide. Antimetabolites (including folic acid antagonists, pyrimidine analogs, purine analogs and adenosine deaminase inhibitors) may include, for example, Methotrexate, 5-Fluorouracil, Floxuridine, Cytarabine, 6-Mercaptopurine, 6-Thioguanine, Fludarabine phosphate, Pentostatine, and Gemcitabine. Natural products and their derivatives (including vinca alkaloids, antitumor antibiotics, enzymes, lymphokines and epipodophyllotoxins) may also be used and include, for example, Vinblastine, Vincristine, Vindesine, Bleomycin, Dactinomycin, Daunorubicin, Doxorubicin, Epirubicin, Idarubicin, paclitaxel (paclitaxel is commercially available as Taxol, Mithramycin, Deoxyco-formycin, Mitomycin-C, L-Asparaginase, Interferons (especially IFN-alpha), Etoposide, and Teniposide. Hormones and steroids (including synthetic analogs) include, for example, 17-alpha-Ethinylestradiol, Diethylstilbestrol, Testosterone, Prednisone, Fluoxymesterone, Dromostanolone propionate, Testolactone, Megestrolacetate, Tamoxifen, Methylprednisolone, Methyltestosterone, Prednisolone, Triamcinolone, Chlorotrianisene, Hydroxyprogesterone, Aminoglutethimide, Estramustine, Medroxyprogesteroneacetate, Leuprolide, Flutamide, Toremifene, or Zoladex. Exemplary synthetics (including inorganic complexes such as platinum coordination complexes) include Cisplatin, Carboplatin, Hydroxyurea, Amsacrine, Procarbazine, Mitotane, Mitoxantrone, Levamisole, and Hexamethylmelamine.

Methods and dosages for the safe and effective administration of most of these chemotherapeutic agents are known to those skilled in the art. In addition, their administration is described in the standard literature. For example, the administration of many of the chemotherapeutic agents is described in the "Physicians' Desk Reference" (PDR), e.g., 1996 edition (Medical Economics Company, Montvale, N.J. 07645-1742, USA), the disclosure of which is incorporated herein by reference.

The following examples are provided for the purpose of illustrating the invention and should not be construed as limiting.

### Example 1

### Materials and Methods

### Producing Hybridomas

We immortalized lymphocytes by fusing them to the HAB-1 heteromyeloma as follows.

We washed the HAB-1 heteromyeloma cells twice with RPMI 1640 (PAA, Vienna, Austria) without additives and centrifuged the cells for 5 minutes at 1500 rpm. We then thawed frozen lymphocytes obtained from either the spleen or the lymph nodes and we washed these cells twice with RPMI 1640 without additives and centrifuged these cells at 1500 rpm for 5 minutes. Both the HAB-1 and the lymphocyte cell pellets were resuspended in 10 ml RPMI 1640 without additives and were counted in a Neubauer cell counting chamber. We washed the cells again, added the HAB-1 cells and the lymphocytes together in a ratio of 1:2 to 1:3, mixed them, and centrifuged the mixture for 8 minutes at 1500 rpm. We pre-warmed Polyethylene Glycol 1500 (PEG) to 37°C and carefully let the PEG run drop-wise onto the pellet while slightly rotating the 50 ml tube. Next, we gently resuspended the pellet and rotated the tube for exactly 90 seconds in a 37°C waterbath. We washed the cells twice with a full 10 ml pipette of RPMI without additives and centrifuged the cells for 5 minutes at 1500 rpm. We added 1 ml of RPMI 1640 with HAT supplement (PAA, Vienna, Austria) and 10% FCS, 1% glutamine, and 1% penicillin/streptomycin ("RPMI 1640 HAT") into each well of a 24-well plate. The cell pellet was dissolved in RPMI 1640 HAT and 0.5 ml of the cells was added to each well of the 24-well plate. We then placed the 24-well plates into a 37°C incubator and changed the RPMI 1640 HAT medium weekly.

Using this protocol, approximately 80% to 90% of the triomas generated are viable and approximately 50% secrete immunoglobulins.

### Sequencing the Antibody

To obtain the sequence of the antibody, we isolated whole RNA from the trioma using the RNASE Kit from Qiagen and we used this RNA to generate cDNA using M-MLV reverse transcriptase (Gibco). After generating the cDNA, the variable region genes were amplified using the polymerase chain reaction (PCR) and Taq polymerase (MBI-Fermentas). The PCR products were purified using gel electrophoresis followed by gel extraction of the PCR product. The PCR products were then cloned using the pCR-Script Amp SK⁺ cloning kit (Stratagene) and the positive clones were sequences using the DyeDeoxy termination cycle sequencing kit (Applied BioSystems). The sequences were analysed using the Dnasis for Windows sequence comparison program, and the GenBank and V-base databases as described in Vollmers et al. (Oncol. Rep. 5:35-40, 1998).

### Immunohistochemical Staining of Paraffin Sections

Paraffin was removed from tissue section by incubating the tissue sections in the following washes:
Two xylene washes for 5 minutes each,
Two 100% ethanol washes for 5 minutes each,
Two 90% ethanol washes for 5 minutes each,
Two 70% ethanol washes for 5 minutes each, and
Three washes in distilled H₂O.

The slides containing the tissue sections were incubated in 75 ml distilled H₂O and 25 ml de-masking solution (Demaskierungslösung G, Biologo, Kronshagen, Germany) in a preheated water-bath at 100°C for 20 minutes. The slides were placed into TrisNaCl (3 grams Tris, 40.5 grams NaCl in 5 litres of distilled H₂O and pH adjusted to 7.4 with HCl) for 5 minutes, blocked for 30 minutes with 150µl of 0.5% Bovine Serum Albumin Fraction V ("BSA"; Roth, Karlsruhe, Germany) in phosphate buffered saline ("PBS") per slide, and washed once with Tris/NaCl.

The slides were incubated with the primary antibody as follows: 150µl of a solution containing the primary antibody, e.g., CM-1 at 25µg/ml in 0.5% BSA/PBS, was added to each microscope slides and the slides were incubated for 2.5 hours in a humidified chamber at 37°C. After the incubation period, the slides were washed three times with Tris/NaCl.

The tissue sections were incubated in the secondary antibody as follows. 150µl of a solution containing the secondary antibody (700µl PBS + 300µl rabbit serum + e.g., 20µl rabbit anti-human IgM antibody; Dako, Hamburg, Germany) was added to each microscope slide. The slides were then incubated for 45 minutes in a humidified chamber at room temperature.

After the incubation period, the slides were washed three times with Tris/NaCl and placed into PBS for 10 minutes. The slides were then incubated for 10 minutes with a solution containing 0.05% diaminobenzidine and 0.02% hydrogen peroxide (Sigma, Taufkirchen (München), Germany). 150µl of this solution was added to each microscope slide. After this incubation period, the slides were washed three times with H₂O, once with distilled H₂O, and placed into hematoxylin solution (Roth, Karlsruhe, Germany) for 5 minutes. The slides were then rinsed for 10-15 min under running tap water, washed with distilled H₂O, and covered with pre-warmed glycerol/gelatine.

Control antibodies used in these assays include the following antibodies: A mouse monoclonal antibody against human cytokeratin 8 ("CK 8"; Cymbus Biotechnology Ltd., Chandlers Ford, Hants, UK), see also, Moll et al. (Cell 31:11-24, 1982); a mouse monoclonal antibody against human cytokeratin 5/6 ("CK 5/6"; Dako A/S, Denmark); and a mouse monoclonal antibody against human cytokeratin ("CAM 5.2"; Becton Dickinson, New Jersey).

### Cytospin Preparation

The adherent growing cells were detached by adding Trypsin/EDTA (PAA, Vienna, Austria) followed by a 5 minute incubation in an humidified incubator (37°C, 5% CO₂) and centrifugation for 5 minutes at 1500 rpm. The cells then were washed twice with 10ml of RPMI-1640 cell culture medium (PAA, Vienna, Austria). The cell number was adjusted to a density of 1 x 10⁵ cells/ml. From this solution, 100µl were centrifuged onto microscope slides with a cytospin centrifuge (CYTOSPIN 2, Shandon, UK) for 2 minutes at 50 rpm. The resultant cytospins were dried for at least 2 hours and stained as specified below.

### Immunoperoxidase Staining of Cytospins and Cryosections

Cytospins were dried for at least two hours at room temperature or cryosections were dried for at least two hours after they were cut. The sections or cytospins were then fixed for 10 minutes in acetone. The fixed cryosections/cytospins were dried for 30 minutes at room temperature, washed three times with Tris-NaCl (3 grams Tris, 40.5 grams NaCl in 5 litres of distilled H₂O and pH adjusted to 7.4 with HCl), and placed into Tris/NaCl for 5 minutes. The cryosections/cytospins were blocked for 15-30 minutes with 3% milk powder in PBS (100µl per cryosection/cytospin) and washed three times with Tris-NaCl. The cryosections/cytospins were incubated in 100µl of primary antibody per cryosection/cytospin (e.g., CM-1 at 20µlg/ml in 0.5% BSA/PBS; CK 8 at 1:50 in BSA/PBS; CAM 5.2 at 1:10 in BSA/PBS; or RPMI 1640 media (PAA, Vienna, Austria) as a negative control) for 30 minutes in a humidified chamber at room temperature. Following the incubation, the cryosections/cytospins were washed three times with Tris-NaCl.

The cryosections/cytospins were then incubated in 100µl of a solution containing the secondary antibody (70 % PBS + 30% rabbit or human serum + e.g., 1:50 rabbit anti-mouse antibody, peroxidase coupled or 1:50 rabbit anti-human IgM antibody, peroxidase coupled; Dako, Hamburg, Germany) per cryosection/cytospin for 30 minutes in a humidified chamber at room temperature and washed three times with Tris-NaCl and placed into PBS for 10 minutes. The cryosections/cytospins where then incubated for 10 minutes in 100 µl of a solution containing 0.05% diaminobenzidine and 0.02% hydrogen peroxide (Sigma, Taufkirchen (Munchen), Germany). Following the incubation, the cryosections/cytospins were washed with distilled H₂O and placed into a hematoxylin staining solution (Roth, Karlsruhe, Germany) for 5 minutes. The cryosections/cytospins were then rinsed for 15 minutes under running tap water, washed with distilled H₂O, and cover with pre-warmed glycerol gelatine.

The following experiments were carried out using the above materials and methods.

### Example 2

### Generation of the Cell Line Expressing the CM-1 Monoclonal Antibody

As described above, we obtained the CM-1 monoclonal antibody expressing hybridoma by fusing lymphocytes obtained from the spleen of a patient having a moderately differentiated colorectal adenocarcinoma (tumor staging T2N0Mx, grade 2) with the heteromyeloma cell line HAB-1 (Faller, et a., Br. J. Cancer 62:595-598, 1990). The resultant cell is a type of hybridoma known as a trioma, as it is the fusion of three cells. Like normal B-lymphocytes, this trioma has to ability to produce antibodies. The specificity of the antibody is determined by the specificity of the original lymphocyte from the patient that was used to generate the trioma.

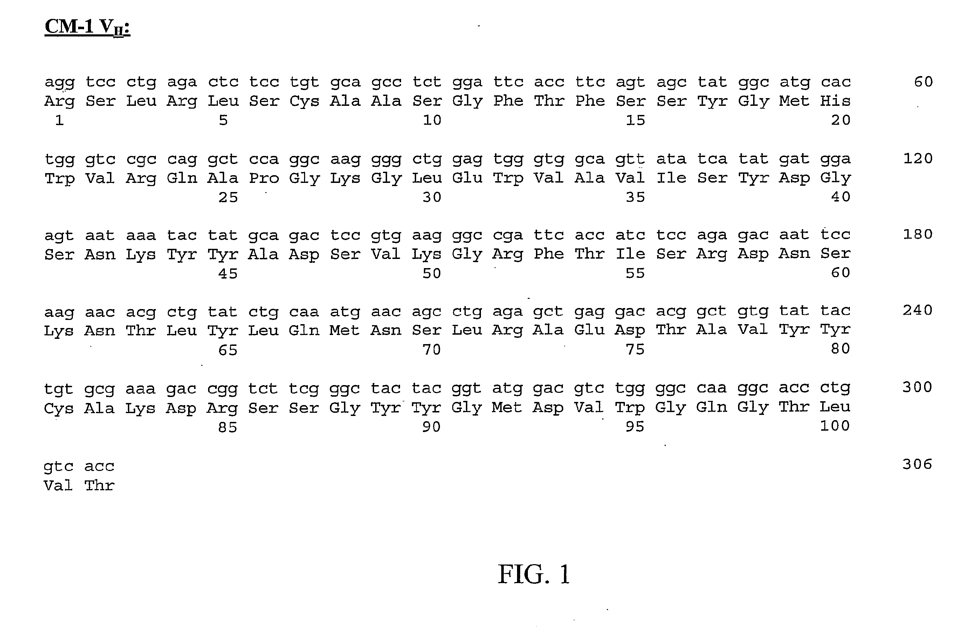

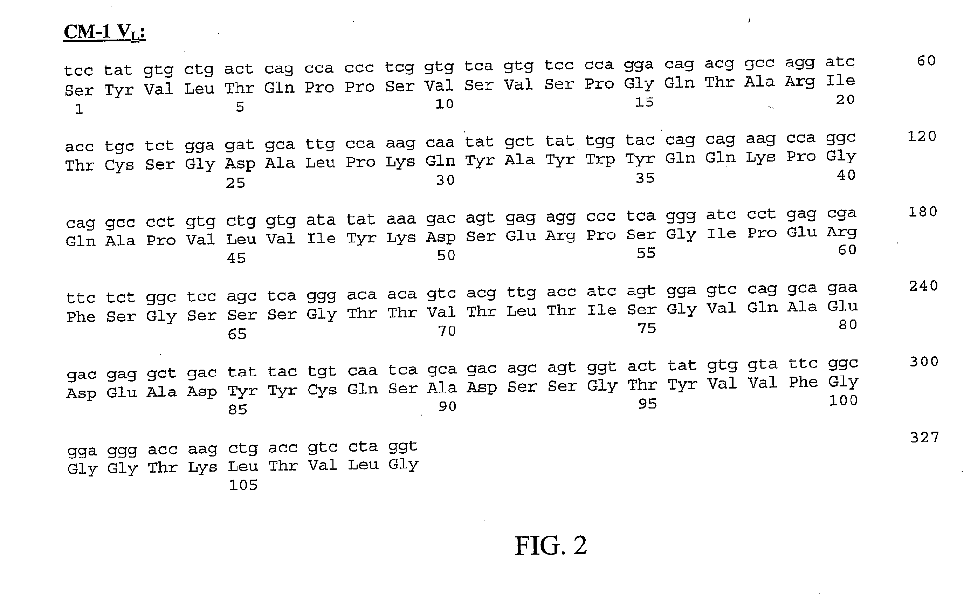

The amino acid and nucleic acid sequences of the CM-1 monoclonal antibody V_{H} region are shown in Figure 1 as SEQ ID NO:1 and SEQ ID NO:2, respectively, and the amino acid and nucleic acid sequences of the CM-1 V_{L} region are shown in Figure 2 as SEQ ID NO:3 and SEQ ID NO:4, respectively. In addition, we compared the sequences of the immunoglobulin chains with germ-line sequences in the International Immunogenetics ("IMGT") database, which is coordinated by Marie-Paule Lefranc at the Université Montpellier, Montpellier, France. We used the DNAPLOT sequence alignment program (available at http://www.dnaplot.org) to identify the most homologous germ-line genes and to detect somatic mutations. The CM-1 V_{H} sequence is homologous to the IGHV3-30/3-30.5*01 germ-line gene (IMGT-No. X92214; Medline No. 88283641; Berman et al., EMBO J. 7:727-738, 1988) and the I_{H} region of the IGHJ5*01 germ-line gene. The CM-1 V_{L} sequence is homologous to the IGLV3-25*03 germ-line gene (IMGT-No. L29165; Medline No. 94216813; Fang et al., J. Exp. Med. 179:1445-1456, 1994) and the I_{L} region of the IGLJ3*01 germ-line gene.

### Example 3

### Immunohistochemical Characterization of an Antibody

To characterize the monoclonal antibody secreted by a hybridoma, we tested the antibody against a panel of normal and tumor tissues using an immunoperoxidase assay as described in the materials and methods. This assay provided us with an overview of which tissues were stained by the antibody and of the distribution of the antigen.

Antibodies that are specific for tumor cells and not for normal tissue were further characterized. First, we tested these antibodies against the same types of tumors from different patients. We then tested these antibodies against tumors of other organs and, finally, against normal tissues. Using these assays, we identified the human CM-1 monoclonal antibody.

The CM-1 monoclonal antibody specifically stains neoplastic cells, such as colorectal carcinoma cells. We stained tissue samples obtained from 21 different colorectal tumors with the CM-1 monoclonal antibody. 10 of these tumors (47.6%) came from female patients and 11 (52.4%) came from male patients. Of the 21 tissue preparations, 20 were adenocarcinomas, of which 2 also contained signet ring cells. In addition, one of the tumors was histologically a squamous cell carcinoma, and three carcinomas were localized in the cecum, two in the sigmoid colon, five in the rectum, and eleven in the other parts of the colon. The staining results are summarized in Table 1, below.

**Table 1:**

| Antibody | Stained | Negative Result | Weak Positive | Strong Positive | Percent Stained |
|---|---|---|---|---|---|
| CM-1 | 21 | 2 | 11 | 8 | 90.5 |

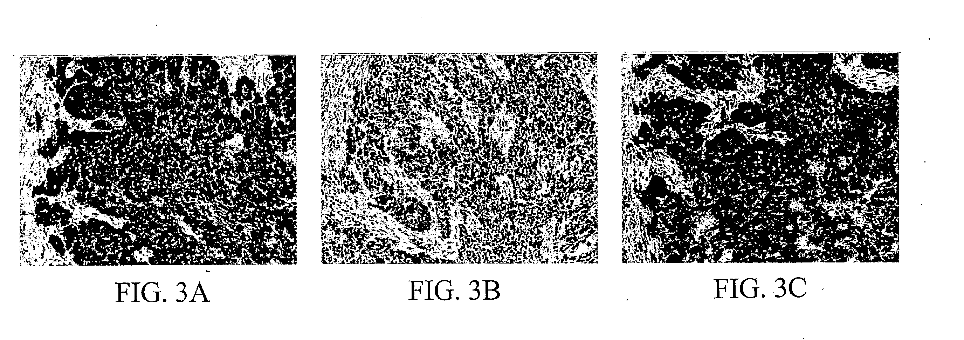

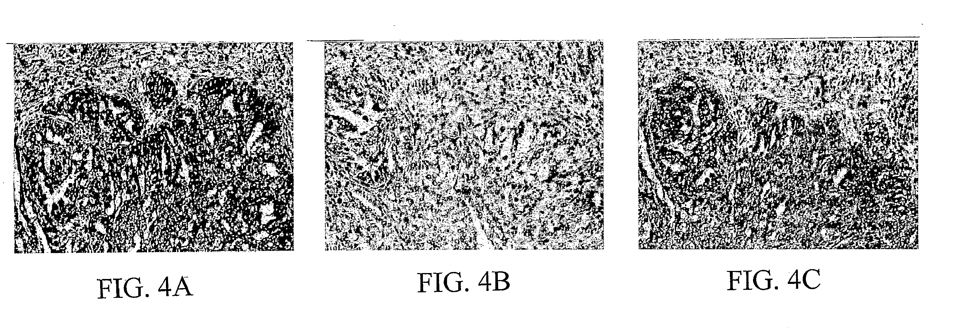

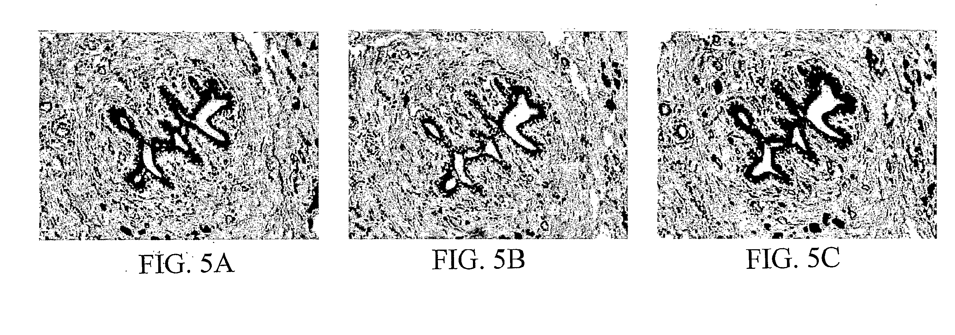

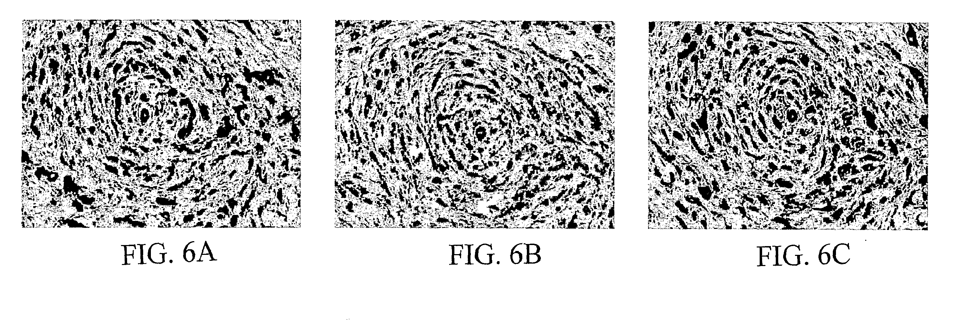

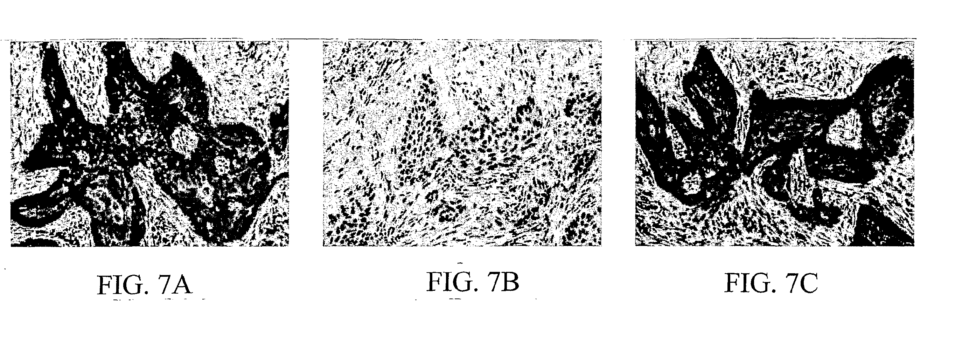

In addition, we determined that the CM-1 antibody specifically stains ovarian adenocarcinomas (Fig. 3A-3C and Fig. 4A-4C), invasive lobular mamma carcinomas (Fig. 5A-5C and Fig. 6A-6C), and squamous cell carcinomas of the lung (Fig. 7A-7C). Furthermore, we determined that the CM-1 antibody also specifically stains stomach carcinoma, esophagial squamous cell carcinoma, pancreatic adenocarcinoma, lung adenocarcinoma, ductal mammary carcinoma, uterine adenocarcinoma, and prostate adenocarcinoma cells.

Moreover, the CM-1 monoclonal antibody also specifically stains a number of colorectal and colon carcinoma cell lines. In particular, the CM-1 antibody binds to the HT-29 human colorectal adenocarcinoma cell line (American Type Culture Collection ("ATCC") Accession No. HTB-38, German Collection of Microorganisms and Cell Cultures ("DSMZ") Accession No. ACC 299), the CACO-2 human colorectal adenocarcinoma cell line (ATCC Accession No. HBT-37, DSMZ Accession No. ACC 169), the human colon carcinoma cell line COLO-320 (DSMZ Accession No. ACC 144), the human colon carcinoma cell line COLO-206F (DSMZ Accession No. ACC 21), and human colon carcinoma cell line COLO-678 (DSMZ Accession No. 194) cells. Slides of these cells were stained according to the cytospin protocol described in the materials and methods section.

### Example 4

### Determining whether an Antibody Induces Apoptosis

A number of assays standard in the art may be used to determine if an antibody induces apoptosis of a cell.

For example, we used the CELL DEATH DETECTION ELISA^{PLUS} (Roche, Mannheim, Germany) to analyze the extent to which the CM-1 antibody induces apoptosis. The cell death detection ELISA is based on a quantitative sandwich-enzyme-immunoassay principle using mouse monoclonal antibodies directed against DNA and histones, respectively. This assay allows the specific determination of mono- and oligo-nucleosomes which are released into the cytoplasm of cells which die from apoptosis.

In particular, we used 100 µl of a cell suspension (1.0 x 10⁵/ml) for each cell line and diluted this one-to-one with 100 µl of the supernatant containing the monoclonal antibody in a 96-well plate and incubated the plate at 37°C and in 7% CO₂ for 24 hours. After the incubation period, the cells were centrifuged at 200 g for 10 minutes, the supernatant was aspirated and 200 µl of the lysis buffer were added, which resulted in the lysis of the cells following a 30 minute incubation at room temperature. After centrifuging again, 20 µl of the supernatant were added to streptavidin-coated mico-titer plates and 80 µl of the immuno-reagent (1/20 Anti-DNA-peroxidase (anti-DNA-POD) antibody which reacts with the DNA components of the nucleosomes, 1/20 Anti-Histone Biotin, 18/20 incubation buffer) were added. In addition, we used the positive control and the blank included in the manufacturers test kit. After the plates were incubated for 2 hours while being mixed at approximately 250 rpm, each well was washed three times with 250 µl of incubation buffer. 100 µl of the ABTS™ solution (1 ABTS™ (2,2'-Azino-di[3-ethyl-benz-thiazolin-sufonat) tablet in 5 ml substrate buffer) were then added to each well. The plates were mixed again and intensity of the antibody-induced apoptosis is reflected in the intensely green precipitate. The color intensity was determined using an ELISA reader at a wavelength of 415 nm against a reference wavelength of 490 nm. Based on this color intensity, we calculated the intensity of the antibody-induced apoptosis.

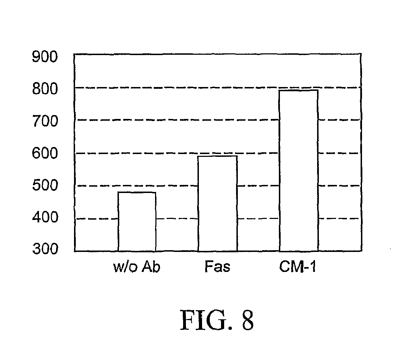

As is shown in Figure 8, CM-1 induces apoptosis of CACO-2 human colorectal carcinoma cells after a 24 hour incubation. The Y-axis in this figure is the difference between the absorbance at 415 nm and at the 490 nm reference wavelength (A₄₁₅-A₄₉₀). The negative control is RPMI 1460 medium. As is shown in Figure 8, both a commercially available CD95 Fas antibody at 2 µg/ml and the supernatant containing the CM-1 monoclonal antibody (45 µg/ml) induce apoptosis when compared to the negative control. The effect seen with the CM-1 monoclonal antibody is 1.46 times that of the negative control.

### Example 5

### Determining whether an Antibody Inhibits Cell Proliferation

Cell proliferation may be assayed by a number of methods that are standard in the art, for example, by the reduction of tetrazolium salts. The yellow tetrazolium salt 3-(4,5-dimethylthiazol-2-yl) - 2,5-diphenyltetrazolium bromide ("MTT") (Sigma, St. Louis, MO), is reduced by metabolically active cells, in part by the action of mitochondrial dehydogenase enzymes to generate reducing equivalents such as NADH and NADPH. The resulting intracellular purple formazan can be solubilized and quantified by spectrophotometric means. The MTT cell proliferation assay measures the rate of cell proliferation and, when metabolic events lead to apoptosis, the reduction in cell viability.

For the MTT assay, we trypsinized cells and resuspended the cells in 10 ml of RPMI-1460 medium contains 10% Fetal Calf Serum (FCS), 1% glutamine, and 1% penicillin/streptomycin (complete medium). The cells were then counted and diluted to 1 x 10⁶ cells/ml. 50 µl of this suspension were pipetted into wells of a 96-well plate, resulting in approximately 5 x 10⁴ cells/well. The first row of wells was left empty. We then added 50 µl of the antibody diluted in complete medium to each well. The 96-well plate was then incubated for 24 or 48 hours in a 37°C incubator. After the incubation period, 50 µl MTT solution (5 mg/ml in PBS) were added to each well. The 96-well plate was incubated for 20 minutes at 37°C and centrifuged for 10 minutes at 2800 rpm. The supernatant was aspirated, 150 µl of DMSO were added to each well, and the cell pellet was resuspended. Absorption was determined at a wavelength of 540 nm and at a reference wavelength of 690 nm in an ELISA reader.

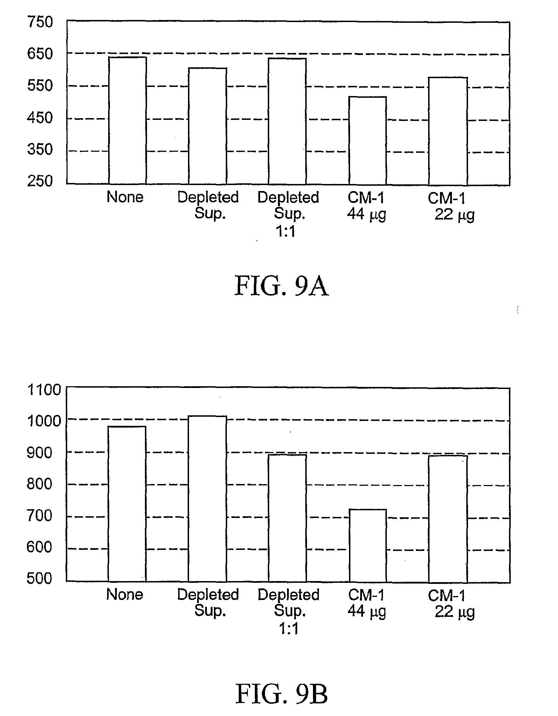

Exemplary results of such experiments depicted in Figures 9A and 9B. Here, COLO-206F human colon carcinoma cells were incubated with the CM-1 monoclonal antibody, with depleted supernatant, or without an antibody for 24 hours (Fig. 9A) or 48 hours (Fig. 9B). The y-axis shows the difference in absorbance at 540 nm and 690 nm (A₅₄₀-A₆₉₀). As is evident from these graphs, the CM-1 antibody, at both 22 µg/ml and 44 µg/ml resulted in a decrease in cell proliferation and cell viability after both a 24 hour and a 48 hour incubation period.

### Example 6

### In Vivo Imaging of a Neoplasm

A patient suspected of having a neoplasm, such as a colorectal carcinoma, maybe given a dose of radioiodinated CM-1 antibody, or another tumor-specific polypeptide, and radiolabeled unspecific antibody using the methods described herein. Localization of the tumor for imaging may be effected according to the procedure of Goldenberg et al. (N. Engl. J. Med., 298:1384, 1978). By I.V. an infusion of equal volumes of solutions of ¹³¹I-CM-1 antibody and Tc-99m-labeled unspecific antibody may be administered to a patient. Prior to administration of the reagents I.V., the patient is typically pretested for hypersensitivity to the antibody preparation (unlabeled) or to antibody of the same species as the antibody preparation. To block thyroid uptake of ¹³¹I, Lugol's solution is administered orally, beginning one or more days before injection of the radioiodinated antibody, at a dose of 5 drops twice or three-times daily. Images of various body regions and views may be taken at 4, 8, and 24 hours after injection of the labeled preparations. If present, the neoplasm, e.g., a colorectal carcinoma, is detected by gamma camera imaging with subtraction of the Tc-99m counts from those of ¹³¹I, as described for ¹³¹I - labeled anti-CEA antibody and Tc- 99m-labeled human serum albumin by DeLand et al. (Cancer Res. 40:3046, 1980). At 8 hours after injection, imaging is usually clear, improving with time up to the 24 hour scans.

### Example 7

### Treatment of a Neoplasm Using Labeled Antibody Mixtures

A patient diagnosed with a neoplasm, for example, a female patient diagnosed with a breast carcinoma, may be treated with the polypeptides of the invention as follows. Lugol's solution may be administered, e.g., 7 drops 3 times daily, to the patient. Subsequently, a therapeutic dose of ¹³¹I-CM-1 antibody may be administered to the patient. For example, a ¹³¹I dose of 50 mCi may be given weekly for 3 weeks, and then repeated at intervals adjusted on an individual basis, e.g., every three months, until hematological toxicity interrupts the therapy. The exact treatment regimen is generally determined by the attending physician or person supervising the treatment. The radioiodinated antibodies may be administered as slow I.V. infusions in 50 ml of sterile physiological saline. After the third injection dose, a reduction in the size of the primary tumor and metastases may be noted, particularly after the second therapy cycle, or 10 weeks after onset of therapy.

### Example 8

### Treatment Using Conjugated Antibodies

A patient diagnosed with a neoplasm, for example, a female patient with breast cancer that has metastasized to the chest and lungs, may be treated with solutions of ¹³¹I-CM-1, ¹⁰B-CM-1, and a Tc-99m labeled unspecific antibody. An amount of ¹³¹I-labeled CM-1 antibody (in 50 ml of sterile physiological saline) sufficient to provide 100 mCi of ¹³¹I activity based on a 70 kg patient weight may be administered to the patient. This dosage is equal to 3.3 mg of an antibody having 40-80 Boron atoms and 8-16 Boron-10 atoms per antibody molecule. The neoplasm is first precisely localized using the procedure of Example 6. In addition, Lugol's solution should be continuously administered to the patient, as in the previous example. A well-collimated beam of thermal neutrons may then be focused on the defined tumor locations. Irradiation with an external neutron beam dose of 400-800 rads, delivered in a period of from 8-20 min, is effected for each tumor locus, and is optionally repeated with administration of the tumor-locating antibody, with or without the radiolabel, at intervals adjusted on an individual basis, but usually not exceeding a total dose of 3200 rads unless simultaneous external irradiation therapy is indicated. If desired, in addition to this therapy, an anti-tumor agent, such as a chemotherapeutic agent, may also be administered to the patient.

### Other Embodiments

While the invention has been described in connection with specific embodiments thereof, it will be understood that it is capable of further modifications and this application is intended to cover any variations, uses, or adaptations of the invention following, in general, the principles of the invention and including such departures from the present disclosure come within known or customary practice within the art to which the invention pertains and may be applied to the essential features hereinbefore set forth.

German patent application no. 102 10 427.1, U.S. Patent Nos. 5,367,060 and 5,641,869 and all other references cited herein are hereby incorporated by reference.

We claim:
1. A purified polypeptide comprising an antibody, or a functional fragment thereof, that induces apoptosis of a neoplastic cell to which it binds, but does not induce apoptosis of a non-neoplastic cell, wherein said antibody specifically binds to at least one of HT-29 (ATCC Accession No. HTB-38; DSMZ Accession No. ACC 299), CACO-2 (ATCC Accession No. HBT-37; DSMZ Accession No. ACC 169), COLO-320 (DSMZ Accession No. ACC 144), COLO-206F (DSMZ Accession No. ACC 21), or COLO-678 (DSMZ Accession No. 194) cells and not to non-neoplastic cells.
2. The purified polypeptide of clause 1, wherein said neoplastic cell is a colorectal adenocarcinoma, ovarian cancer, squamous cell lung carcinoma, or lobular mammary carcinoma cell.
3. A purified polypeptide comprising an antibody, or a functional fragment thereof, that induces apoptosis of a neoplastic cell to which it binds, but does not induce apoptosis of a non-neoplastic cell, wherein said antibody specifically binds to a colorectal adenocarcinoma, ovarian cancer, squamous cell lung carcinoma, or lobular mammary carcinoma cell and not to a non-neoplastic cell.
4. A purified polypeptide comprising an antibody, or a functional fragment thereof, that inhibits cell proliferation when bound to a neoplastic cell, but does not inhibit cell proliferation of a non-neoplastic cell, wherein said antibody specifically binds to at least one of HT-29 (ATCC Accession No. HTB-38; DSMZ Accession No. ACC 299), CACO-2 (ATCC Accession No. HBT-37; DSMZ Accession No. ACC 169), COLO-320 (DSMZ Accession No. ACC 144), COLO-206F (DSMZ Accession No. ACC 21), or COLO-678 (DSMZ Accession No. 194) cells and not to non-neoplastic cells.
   clause
5. The purified polypeptide of clause 4, wherein said neoplastic cell is a colorectal adenocarcinoma, ovarian cancer, squamous cell lung carcinoma, or lobular mammary carcinoma cell.
6. A purified polypeptide comprising an antibody, or a functional fragment thereof, that inhibits cell proliferation when bound to a neoplastic cell, but does not inhibit cell proliferation of a non-neoplastic cell, wherein said antibody specifically binds to a colorectal adenocarcinoma, ovarian cancer, squamous cell lung carcinoma, or lobular mammary carcinoma cell and not to a non-neoplastic cell.
7. The purified polypeptide of clauses 1, 3, 4, or 6, wherein said polypeptide comprises a sequence that is substantially identical to the amino acid sequence of SEQ ID NO:1.
8. The purified polypeptide of clause 1, 3, 4, or 6, wherein said polypeptide comprises a sequence that is substantially identical to the amino acid sequence of SEQ ID NO:3.
9. A purified polypeptide comprising an antibody, or functional fragment thereof, wherein said polypeptide comprises the amino acid sequence of SEQ ID NO:1.
10. A purified polypeptide comprising an antibody, or functional fragment thereof, wherein said polypeptide comprises the amino acid sequence of SEQ ID NO:3.
11. A purified polypeptide comprising an antibody, or functional fragment thereof, wherein said polypeptide comprises the amino acid sequence of SEQ ID NOS:1 and 3.
12. The purified polypeptide of clause 1, 3, 4, 6, 9,10, or 11, wherein said antibody is a human antibody.
13. The purified polypeptide of clause 1, 3, 4, 6, 9,10, or 11, wherein said antibody is a monoclonal antibody.
14. The purified polypeptide of clause 1,3,4,6,9,10, or 11, wherein said polypeptide is a functional fragment selected from the group consisting of V_{L}, V_{H}, F_{V}, F_{C}, Fab, Fab', and F(ab')₂.
15. The purified polypeptide of clause 1, 3, 4, 6, 9, 10, or 11, wherein said polypeptide is a functional fragment comprising a fragment that is substantially identical to the sequence of SEQ ID NO:1 or SEQ ID NO:3.
   clause
16. The purified polypeptide of clause 1,3, 4, 6, 9, 10, or 11, wherein said polypeptide is a functional fragment comprising a fragment of the sequence of SEQ ID NO:1 or SEQ ID NO:3.
17. A cell that produces a polypeptide that induces apoptosis of a neoplastic cell to which it binds, but does not induce apoptosis of a non-neoplastic cell, wherein said polypeptide specifically binds to at least one of HT-29 (ATCC Accession No. HTB-38; DSMZ Accession No. ACC 299), CACO-2 (ATCC Accession No. HBT-37; DSMZ Accession No. ACC 169), COLO-320 (DSMZ Accession No. ACC 144), COLO-206F (DSMZ Accession No. ACC 21), or COLO-678 (DSMZ Accession No. 194) cells and not to non-neoplastic cells.
18. The cell of clause 17, wherein said neoplastic cell is a colorectal adenocarcinoma, ovarian cancer, squamous cell lung carcinoma, or lobular mammary carcinoma cell.
19. A cell that produces a polypeptide that inhibits cell proliferation in a neoplastic cell to which it binds, but not in a non-neoplastic cell, wherein said polypeptide specifically binds to at least one of HT-29 (ATCC Accession No. HTB-38; DSMZ Accession No. ACC 299), CACO-2 (ATCC Accession No. HBT-37; DSMZ Accession No. ACC 169), COLO-320 (DSMZ Accession No. ACC 144), COLO-206F (DSMZ Accession No. ACC 21), or COLO-678 (DSMZ Accession No. 194) cells and not to non-neoplastic cells.
20. The cell of clause 19, wherein said neoplastic cell is a colorectal adenocarcinoma, ovarian cancer, squamous cell lung carcinoma, or lobular mammary carcinoma cell.
21. A cell that produces a polypeptide that comprises a sequence that is substantially identical to the amino acid sequence of SEQ ID NO:1.
   clause
22. The cell of clause 21, wherein said polypeptide comprises the sequence of SEQ ID NO:1.
23. A cell that produces a polypeptide that comprises a sequence that is substantially identical to the amino acid sequence of SEQ ID NO:3.
24. The cell of clause 23, wherein said polypeptide comprises the sequence of SEQ ID NO:3.
25. A cell that produces a polypeptide that comprises the amino acid sequence of SEQ ID NOS:1 and 3.
26. The cell of clause 17, 19, 21, 23, or 25, wherein said cell is a hybridoma.
27. A method of generating the cell of clause 17, said method comprising the steps of:
   (a) contacting lymphocytes with a heteromyeloma cell line under conditions that result in the fusion of a lymphocyte with a heteromyeloma cell, said fusion resulting in a hybridoma,
   (b) determining whether said hybridoma produces a polypeptide that induces apoptosis of a neoplastic cell to which it binds, but does not induce apoptosis of a non-neoplastic cell, and
   (c) determining whether said hybridoma produces polypeptide that specifically binds to at least one of HT-29 (ATCC Accession No. HTB-38; DSMZ Accession No. ACC 299), CACO-2 (ATCC Accession No. HBT-37; DSMZ Accession No. ACC 169), COLO-320 (DSMZ Accession No. ACC 144), COLO-206F (DSMZ Accession No. ACC 21), or COLO-678 (DSMZ Accession No. 194) cells and not to non-neoplastic cells.
28. A method of generating the cell of clause 19, said method comprising the steps of:
   (a) contacting lymphocytes with a heteromyeloma cell line under conditions that result in the fusion of a lymphocyte with a heteromyeloma cell, said fusion resulting in a hybridoma,
   (b) determining whether said hybridoma produces a polypeptide that inhibits proliferation in a neoplastic cell to which it binds, but does not inhibit proliferation in a non-neoplastic cell, and
   (c) determining whether said hybridoma produces polypeptide that specifically binds to at least one of HT-29 (ATCC Accession No. HTB-38; DSMZ Accession No. ACC 299), CACO-2 (ATCC Accession No. HBT-37; DSMZ Accession No. ACC 169), COLO-320 (DSMZ Accession No. ACC 144), COLO-206F (DSMZ Accession No. ACC 21), or COLO-678 (DSMZ Accession No. 194) cells and not to non-neoplastic cells.
29. Use of the purified polypeptide of clause 1, 3, 4, 6, 9, 10, or 11 in a method of diagnosing a neoplasm in a mammal, said method comprising the steps of:
   (a) contacting a cell or tissue sample of said mammal with the purified polypeptide of clause 1, 3, 4, 6, 9, 10, or 11, and
   (b) detecting whether said purified polypeptide binds to said cell or tissue sample, wherein binding of said purified polypeptide to said cell or tissue sample is indicative of said mammal having a neoplasm.
30. The use of cleuse 29, wherein said mammal is a human.
31. The use of clause 29, wherein said neoplasm is a colorectal adenocarcinoma, an ovarian cancer, a squamous cell lung carcinoma, or a lobular mammary carcinoma.
32. The use of clause 29, wherein said polypeptide is an antibody.
33. The use of clause 29, wherein said polypeptide is conjugated to a detectable agent selected from the group consisting of a radionuclide, a fluorescent marker, an enzyme, a cytotoxin, a cytokine, and a growth inhibitor.
34. The use of claim 29, wherein said polypeptide is conjugated to a protein purification tag.
35. The use of clause 34, wherein said protein purification tag is cleavable.
36. Use of the purified polypeptide of clause 1, 3, 4, 6, 9, 10, or 11 in a method of treating a proliferative disorder in a mammal, said method comprising the step of contacting a cell or tissue sample with the purified polypeptide of clause 1, 3, 4, 6, 9, 10, or 11, wherein binding of said purified polypeptide to said cell or tissue sample results in the induction of apoptosis of said cell or tissue sample.
   clause
37. The use of clause 36, wherein said mammal is a human.
38. The use of clause 36, wherein said proliferative disorder is a colorectal adenocarcinoma, an ovarian cancer, a squamous cell lung carcinoma, or a lobular mammary carcinoma.
39. The use of clause 36, wherein said polypeptide is an antibody.
40. The use of clause 36, wherein said polypeptide is conjugated to a detectable agent selected from the group consisting of a radionuclide, a fluorescent marker, an enzyme, a cytotoxin, a cytokine, and a growth inhibitor.
41. The use of clause 40, wherein said detectable agent is capable of inducing apoptosis of said cell or tissue sample.
42. The use of clause 36, wherein said polypeptide is conjugated to a protein purification tag.
43. The use of clause 42, wherein said protein purification tag is cleavable.
44. Use of the purified polypeptide of clause 1, 3, 4, 6, 9, 10, or 11 in a method of treating a proliferative disorder in a mammal, said method comprising the step of contacting a cell or tissue sample with the purified polypeptide of clause 1, 3, 4, 6, 9, 10, or 11, wherein binding of said purified polypeptide to said cell or tissue sample results in a reduction in proliferation of said cell or of a cell in said tissue sample.
45. The use of clause 44, wherein said mammal is a human.
46. The use of clause 44, wherein said proliferative disorder is a colorectal adenocarcinoma, an ovarian cancer, a squamous cell lung carcinoma, or a lobular mammary carcinoma.
47. The use of clause 44, wherein said polypeptide is an antibody.
48. The use of clause 44, wherein said polypeptide is conjugated to a detectable agent selected from the group consisting of a radionuclide, a fluorescent marker, an enzyme, a cytotoxin, a cytokine, and a growth inhibitor.
49. The use of clause 48, wherein said detectable agent is capable of inhibiting cell proliferation of said cell or tissue sample.
50. The use of clause 44, wherein said polypeptide is conjugated to a protein purification tag.
51. The use of clause 50, wherein said protein purification tag is cleavable.
52. A medicament comprising the purified polypeptide of any one of clauses 1, 3, 4, 6, 9, 10, or 11 in a pharmaceutically acceptable carrier.
53. A diagnostic agent comprising the purified polypeptide of any one of clauses 1, 3, 4, 6, 9, 10, or 11.

## Claims

1. A purified antibody, or a functional fragment thereof, wherein said antibody or functional fragment specifically binds to at least one of HT-29 (ATCC Accession No. HTB-38; DSMZ Accession No. ACC 299), CACO-2 (ATCC Accession No. HBT-37; DSMZ Accession No. ACC 169), COLO-320 (DSMZ Accession No. ACC 144), COLO-206F (DSMZ Accession No. ACC 21), or COLO-678 (DSMZ Accession No. 194) cells, and wherein said antibody or functional fragment thereof comprises a heavy chain variable region sequence that is at least 75% identical to SEQ ID NO:1 and a light chain variable region sequence that is at least 75% identical to SEQ ID NO:3.

2. (New) A purified antibody, or a functional fragment thereof, wherein said antibody or functional fragment specifically binds to a colorectal adenocarcinoma, ovarian cancer, squamous cell lung carcinoma, or lobular mammary carcinoma cell, and wherein said antibody or functional fragment thereof comprises a heavy chain variable region sequence that is at least 75% identical to SEQ ID NO:1 and a light chain variable region sequence that is at least 75% identical to SEQ ID NO:3.

3. The purified antibody, or a functional fragment thereof of claims 1 or 2, wherein said antibody or functional fragment comprises a heavy chain variable region sequence that is at least 80% identical to SEQ ID NO:1 or a light chain variable region sequence that is at least 80% identical to SEQ ID NO:3.

4. The purified antibody, or functional fragment thereof of any of claims 1 to 3, wherein said antibody or functional fragment comprises a heavy chain variable region sequence that is at least 90% identical to SEQ ID NO:1 or a light chain variable region sequence that is at least 90% identical to SEQ ID NO:3.

5. The purified antibody or functional fragment of any of claims 1 to 4, wherein said antibody or functional fragment is human.

6. The purified antibody or functional fragment of any of claims 1 to 4, wherein said antibody or functional fragment is monoclonal.

7. The purified antibody or functional fragment of any of claims 1 to 6, wherein said functional fragment is selected from the group consisting of V_{L}, V_{H}, F_{V}, Fab, Fab', and F(ab')2.

8. The antibody or functional fragment of any of claims 1 to 7, wherein said antibody or functional fragment induces apoptosis or inhibits proliferation of at least one of HT-29 (ATCC Accession No. HTB-38; DSMZ Accession No. ACC 299), CACO-2 (ATCC Accession No. HBT-37; DSMZ Accession No. ACC 169), COLO-320 (DSMZ Accession No. ACC 144), COLO-206F (DSMZ Accession No. ACC 21), or COLO-678 (DSMZ Accession No. 194) cells.

9. A cell that produces an antibody or functional fragment of any of claims 1 to 8.

10. A cell that produces a heavy chain variable region sequence that is at least 75% identical to SEQ ID NO:1 or a light chain variable region sequence that is at least 75% identical to SEQ ID NO:3.

11. A method of generating an antibody or functional fragment of any of claims 1 to 8, comprising expressing nucleic acid sequences encoding the antibody or functional fragment of any of claims 1 to 8 in a host cell.

12. Use of the antibody or functional fragment any of claims 1 to 8 in a method of diagnosing a neoplasm in a mammal, said method comprising the steps of:
(a) contacting a cell or tissue sample of said mammal with said purified antibody or functional fragment; and
(b) detecting whether said purified antibody or functional fragment binds to said cell or tissue sample, wherein binding of said purified antibody or functional fragment to said cell or tissue sample is indicative of said mammal having a neoplasm.

13. Use of the purified antibody or functional fragment of claim 1 to 8 for the manufacture of a medicament for treating a proliferative disorder in a mammal, wherein binding of said purified antibody or functional fragment to a cell or tissue results in the induction of apoptosis of said cell or tissue.

14. Use of the purified antibody or functional fragment of any of claims 1 to 8 in a method of treating a proliferative disorder in a mammal, wherein binding of said purified antibody or functional fragment to a cell or tissue results in a reduction in proliferation of said cell or of a cell in said tissue.

15. The use of claims 13 or 14, wherein said neoplasm or said proliferative disorder is a colorectal adenocarcinoma, an ovarian cancer, a squamous cell lung carcinoma, or a lobular mammary carcinoma.

16. The use of claims 13 or 14, wherein said antibody or functional fragment is conjugated to a detectable agent selected from the group consisting of a radionuclide, a fluorescent marker, an enzyme, a cytotoxin, a cytokine, and a growth inhibitor.

17. The use of claims 13 or 14, wherein said mammal is a human.

18. A medicament comprising the purified antibody or functional fragment of any one of claims 1 to 8 in a pharmaceutically acceptable carrier.

19. A diagnostic agent comprising the purified antibody or functional fragment of any one of claims 1 to 8.

20. The antibody or functional fragment of any of claims 1 to 8, wherein said antibody or functional fragment has an insertion, deletion or substitution of an amino acid residue in heavy chain variable region sequence set forth as SEQ ID NO:1 or light chain variable region sequence set forth as SEQ ID NO:3.
